# EUROPÄISCHE PATENTSCHRIFT

(11) **EP 1 641 498 B1**
(45) Veröffentlichungstag und Bekanntmachung des Hinweises auf die Patenterteilung: **04.09.2013**
(21) Anmeldenummer: 04740510.5
(22) Anmeldetag: 01.07.2004
(51) Int. Cl.: A61L 15/44, A61L 15/60

(54) **WIRKSTOFFDOTIERTE ABSORBIERENDE POLYMERTEILCHEN, ZUSAMMENSETZUNG BEINHALTEND POLYKONDENSATMATRIX UND ABSORBIERENDES POLYMER ZUR FREISETZUNG EINER WUNDHEILSUBSTANZ**
ABSORBENT POLYMER PARTICLES DOPED WITH ACTIVE INGREDIENTS, COMPOSITION CONTAINING POLYCONDENSATE MATRIX, AND ABSORBENT POLYMER FOR RELEASING A WOUND HEALING SUBSTANCE
PARTICULES POLYMERES ABSORBANTES DOPEES EN PRINCIPE ACTIF, COMPOSITION CONTENANT UNE MATRICE DE POLYCONDENSAT ET POLYMERE ABSORBANT DESTINE A LIBERER UNE SUBSTANCE CICATRISANTE

(30) Priorität: 08.07.2003 DE 10330960
(43) Veröffentlichungstag der Anmeldung: 05.04.2006
(73) Patentinhaber: Evonik Degussa GmbH, 45128 Essen (DE)
(72) Erfinder: HARREN, Jörg, 47807 Krefeld (DE); NIELINGER, Ursula, 47802 Krefeld (DE); OPPENBERG, Dieter, 45468 Mühlheim/Ruhr (DE); SCHMIDT, Harald, 47918 Tönisvorst (DE)
(74) Vertreter: Lang, Arne
(86) Internationale Anmeldenummer: PCT/EP2004/007140
(87) Internationale Veröffentlichungsnummer: WO 2005/004935

(56) Entgegenhaltungen:
- DE-A- 4 233 289
- DE-A- 10 043 710
- DE-A- 10 257 002
- US-A1- 2003 004 479

## Beschreibung

Die vorliegende Erfindung betrifft wirkstoffdotierte wasserabsorbierende Polymerteilchen, eine wasserabsorbierende Zusammensetzung, ein Verfahren zur Herstellung einer wasserabsorbierenden Zusammensetzung, eine durch dieses Verfahren erhältliche wasserabsorbierende Zusammensetzung, einen diese wasserabsorbierende Zusammensetzung beinhaltenden Verbund, die Verwendung dieser wasserabsorbierenden Zusammensetzung oder eines in der wasserabsorbierenden Zusammensetzung beinhalteten wasserabsorbierenden Polymers zur Herstellung eines Mittels zur Freisetzung einer Wundheilsubstanz, die Verwendung dieser wasserabsorbierenden Zusammensetzung oder eines in der wasserabsorbierenden Zusammensetzung beinhalteten wasserabsorbierenden Polymers zur Herstellung eines Mittels zur Behandlung von Wunden, sowie die Verwendung dieser wasserabsorbierenden Zusammensetzung oder eines in der wasserabsorbierenden Zusammensetzung beinhalteten wasserabsorbierenden Polymers in einem Hygieneartikel oder Wundbehandlungsmittel.

Aus dem Stand der Technik sind eine Reihe von Schriften bekannt, in denen wirkstoffdotierte wasserabsorbierende Polymerteilchen offenbart sind. So beschreibt US 2003/0004479 A1 eine wasserabsorbierende Zusammensetzung bestehend aus einem wasserabsorbierenden Polymer und einem pflanzlichen Pulver als Wirlcstoff, wobei das wasserabsorbierende Polymer im Oberflächenbereich nachvernetzt ist. Diese Zusammensetzung ist wegen des enthaltenen Wirkstoffes in der Lage, unangenehme Gerüche zu binden.

DE 101 42 918 A1 offenbart eine selbstklebende Gelmatrix auf nichtneutralisiertem Polyacrylatbasis, die von Wirkstoffen zur kosmetischen oder pharmazeutischen Behandlung der Haut oder systemischen Verabreichung von Arzneimitteln dotiert werden kann. Da die Gelmatrix als zusammenhängende Schicht auf ein Substrat aufgetragen ist, ist die Wirkstoffabgabe dadurch eingeschränkt.

DE 102 57 002 A1 offenbart ein Hautpflegemittel enthaltende schaumförmige Hydrogele auf Basis von vernetzten, Säuregruppen enthaltenden schaumförmigen Polymeren. Die Polymere, die als flächenförmige Gebilde vorgesehen sind, enthalten ein Hautpflegemittel und werden in Hygieneartikeln verwendet. Die Gelmatrix lässt sich nicht ohne erhebliche und damit kostspielige Änderungen der Konstruktion in herkömmliche für wasserabsorbierende Polymerteilchen vorgesehene Hygieneartikel oder Wundauflagen einarbeiten.

Aus dem Stand der Technik sind außerdem eine Reihe von Schriften bekannt, die die Kombination von wasserabsorbierenden Polymeren und Polyurethanen in einer Matrix und deren Einsatz in Wundauflagen und Wundpflastern offenbaren.

So offenbart EP 0 196 364 A2 hydrophile Polyurethanschäume, die mit wasserabsorbierenden Polymeren auf Basis eines Acrylsäure-Kaliumsalz-Polymers gefüllt sind und für medizinische Zwecke eingesetzt werden können.

Weiterhin lehrt DE 42 33 289 A1 hydrophile Polyurethangelschäume mit wasserabsorbierenden Polymeren, die in der Medizin verwendet werden können.

Daher bestehen allgemein die erfindungsgemäßen Aufgaben darin, die sich aus dem Stand der Technik ergebenden Nachteile zumindest abzumildern oder gar zu überwinden.

Insbesondere liegt eine Aufgabe der vorliegenden Erfindung darin, eine wasserabsorbierende Zusammensetzung oder ein diese wasserabsorbierende Zusammensetzung beinhaltendes Mittel zur Verfügung zu stellen, die in der Lage ist, die Haut zu pflegen und die Widerstandskraft der Haut zu erhöhen. Dieses gilt insbesondere für das Entstehen von wunden Bereichen. Diese treten bei Trägem von Windeln durch Langzeitkontakt mit Exkrementen und/oder bei langem Liegen auf Weiterhin ist eine Stärkung der Widerstandskraft der Haut bei Inkontinenzartikel tragenden, bettlegrigen Patienten vorteilhaft, da dadurch der Gefahr des Wundliegens vorgebeugt wird.

Zudem ist eine erfindungsgemäße Aufgabe darin zu sehen, die Haut zu pflegen oder das Abheilen von Wunden zu beschleunigen bzw. eine hierzu geeignete wasserabsorbierende Zusammensetzung oder ein diese wasserabsorbierende Zusammensetzung enthaltendes hierzu geeignetes Mittel zur Verfügung zu stellen.

Weiterhin besteht eine erfindungsgemäße Aufgabe darin, den Tragekomfort von Wundauflagen, von Hautpflastern, insbesondere Wundpflastern oder von Hygieneartikeln zu verbessern und insbesondere das Verlagern und Verrutschen von partikulären wasserabsorbierenden Polymeren in Wundauflagen, in Hautpflastern, insbesondere in Wundpflastern oder in Hygieneartikeln oder deren zur Flüssigkeitsaufuahme bestimmten Bestandteilen zu verhindern, ohne dass die Pflege- und Heilungseigenschaften der Haut- oder Wundpflaster dadurch nachteilig beeinflusst werden.

Außerdem liegt eine erfindungsgemäße Aufgabe darin, bei Wundauflagen, bei Hautpflastern, insbesondere bei Wundpflastern, oder bei Hygieneartikeln das Flüssigkeits-Management so zu gestalten, dass die Wundauflagen, Hautpflaster oder Hygieneartikel über möglichst lange Zeiträume eingesetzt werden können, ohne dass es zu Feuchtigkeits- oder gar Nässestauungen kommt und die Pflege-, Wundvorbeuge- bzw. Heilungswirkung der Wundauflagen, Hautpflaster oder Hygieneartikel nachteilig beeinflusst wird.

Ferner besteht eine erfindungsgemäße Aufgabe darin, dass die Verfügbarkeit einer Wirksubstanz, vorzugsweise einer Wundheilsubstanz oder einer Pflegesubstanz, die in einer Trägermatrix bei der Bildung dieser Trägermatrix eingearbeitet wird, weniger stark durch die durch die Einarbeitung bedingte Zerstörung der Wirksubstanz vermindert wird. Die Gefahr der Verringerung der Wirksamkeit durch Zerstörung der Wirksubstanz besteht insbesondere, wenn diese durch die zur Herstellung der Matrix verwendeten Verbindungen chemisch verändert wird. Diese Gefahr besteht insbesondere, wenn die Matrix aus einem Polymer besteht, dass auf Monomeren mit funktionellen Gruppen basiert, die mit funktionellen Gruppen der Wirksubstanz reagieren können, wobei die Wirksamkeit oder Verfügbarkeit der Wirksubstanz vermindert wird. Dieses lässt sich insbesondere bei der Einarbeitung von Wirksubstanzen in eine Polykondensatmatrix beobachten, wobei vor allem die Isocyanat-Gruppen mit den funktionellen Gruppen der Wirksubstanz reagieren.

Zudem besteht eine erfindungsgemäße Aufgabe darin, Hygieneartikel bereit zu stellen, die bei hohem Tragekomfort und Saugleistungen die Bildung von Wunden und wunden Hautpartien, insbesondere hervorgerufen durch Wundlegen oder ungeeignete Ernährung, vorzubeugen oder diese zu lindern und deren Abheilung zu befördern. Eine weitere erfindungsgemäße Aufgabe bestand darin, Hygieneartikel bereit zu stellen, die bei hohem Tragekomfort und Saugleistungen in der Lage sind, die mit dem Hygieneartikel in Kontakt tretende Haut zu pflegen.

Die vorstehenden Aufgaben werden durch den Gegenstand der Ansprüche und wie nachfolgend ausgeführt gelöst.

Die Erfindung betrifft zur Lösung der Aufgaben wirkstoffdotierte wasserabsorbierende Polymerteilchen, beinhaltend
Φ1. als Wirkstoff eine Pflegesubstanz, insbesondere eine Hautpflegesubstanz, oder eine Wundheilsubstanz, oder deren Salz in einer Menge im Bereich von 0,001 bis 30 Gew.-%, vorzugsweise von 0,1 bis 20 Gew.-% und besonders bevorzugt von 1 bis 15 Gew.-%, jeweils bezogen auf die wirkstoffdotierten wasserabsorbierende Polymerteilchen,
Φ2. eine Absorbermatrix in einer Menge im Bereich von 70 bis 99,999 Gew.-%, vorzugsweise von 80 bis 99,9 Gew.-% und besonders bevorzugt von 90 bis 99 Gew.-%, jeweils bezogen auf die wirkstoffdotierten wasserabsorbierende Polymerteilchen,
wobei die Absorbermatrix zu mindestens 90 Gew.-%, bezogen auf die Absorbermatrix, aus einer vernetzten Polyacrylsäure besteht,
wobei die vernetzte Polyacrylsäure zu mindestens 90 Gew.-%, bezogen auf die vernetzte Polyacrylsäure, aus einer zu mindestens 30 Mol-% teilneutralisierten Acrylsäure besteht.

Zudem ist es in den erfindungsgemäßen wirkstoffdotierten wasserabsorbierenden Polymerteilchen bevorzugt, dass der Wirkstoff über mindestens 70, vorzugsweise mindestens 80, besonders bevorzugt mindestens 90 Vol.-% und darüber hinaus bevorzugt über die gesamte Absorbermatrix verteilt ist. Hierbei ist es besonders bevorzugt, dass der Wirkstoff homogen über die gesamte Absorbermatrix verteilt ist.

Weiterhin zeigen die wirkstoffdotierten wasserabsorbierenden Polymerteilchen in einer bevorzugten Ausführungsform einen Gehalt an restlichem Monomeren, auf dem die wasserabsorbierenden Polymerteilchen basieren, unter 500 ppm, vorzugsweise unter 300 ppm, ferner bevorzugt unter 200 ppm und darüber hinaus bevorzugt unter 146 ppm, jeweils bezogen auf die wasserabsorbierenden Polymerteilchen. Der Restmonomergehalt wird nach ERT 410.1-99 bestimmt.

Ferner zeigen die wirkstoffdotierten wasserabsorbierenden Polymerteilchen in einer anderen Ausführungsform eine Wirkstoffverfügbarkeit von mindestens 40 Gew.-%, vorzugsweise mindestens 70 Gew.-%, besonders bevorzugt mindestens 85 Gew.-% und darüber hinaus bevorzugt mindestens 95 Gew.-% eines in den wasserabsorbierenden Polymerteilchen enthaltenen Wirkstoffes, bestimmt nach dem hierin beschriebenen Extraktions-Test.

Ferner ist es bevorzugt, dass die erfindungsgemäßen wirkstoffdotierten wasserabsorbierenden Polymerteilchen eine Partikelgrößenverteilung aufweisen, wobei mindestens 80 Gew.-% der Partikel eine nach ERT 420.1-99 bestimmte Partikelgröße in einem Bereich von 20 µm bis 900 µm, vorzugsweise im Bereich von 150 µm bis 600 µm und besonders bevorzugt im Bereich von 200 µm bis 400µm besitzen.

Außerdem ist es bevorzugt, dass die erfindungsgemäßen wirkstoffdotierten wasserabsorbierenden Polymerteilchen weniger als 15 Gew.-%, bevorzugt weniger als 10 Gew.-% und besonders bevorzugt weniger als 5 Gew.-%, bezogen auf die wirkstoffdotierten wasserabsorbierenden Polymerteilchen, Teichen einer Teilchengröße von < 100 µm, vorzugsweise < 130 µm und besonders bevorzugt < 150 µm aufweisen.

Weiterhin ist es bevorzugt, dass die erfindungsgemäßen wirkstoffdotierten wasserabsorbierenden Polymerteilchen weniger als 15 Gew.-%, bevorzugt weniger als 10 Gew.-% und besonders bevorzugt weniger als 5 Gew.-%, bezogen auf die wirkstoffdotierten wasserabsorbierenden Polymerteilchen, Teichen einer Teilchengröße von > 950 µm, vorzugsweise > 850 µm und besonders bevorzugt > 650 µm aufweisen.

Die vorstehenden Teilchengrößenverteilungen und Teilchengrößen sind insbesondere für eine gleichmäßige Abgabe und Verteilung des Wirkstoffes und für einen guten Tragekomfort vorteilhaft. Außerdem hat sich gezeigt, dass die vorstehenden Teilchengrößenverteilungen und Teilchengrößen sich besonders gut in flexible Matrices einarbeiten lassen, die, wenn in Pflaster oder Wundauflagen eingearbeitet, die Anpassungsfähigkeit dieser Pflaster oder Wundauflagen an die Haut, insbesondere bei Wundpflastern an die Form der Wunde und an deren Bewegungen erhöht.

Als Pflegesubstanz werden vorzugsweise Hautpflegesubstanzen verstanden, die
a) die Haut reinigen,
b) die Haut parfümieren,
c) das Aussehen der Haut verändern,
d) die Haut schützen oder
e) die Haut in gutem Zustand halten.

Besonders bevorzugt wird unter einem Pflegemittel ein Mittel verstanden, welches mindestens eine, vorzugsweise mindestens zwei, besonders bevorzugt mindestens drei der vorstehend genannten Eigenschaften a) bis e) aufweist. Bevorzugte Ausführungsformen der erfindungsgemäßen wirkstoffdotierten wasserabsorbierenden Polymerteilchen sind weiterhin dadurch gekennzeichnet, dass die Pflegesubstanzen durch folgende Eigenschaften bzw. Eigenschaftskombinationen gekennzeichnet sind: a, b, c, d, e, ab, ac, ad, ae, bc, bd, be, cd, ce, de, abc, abd, abe, acd, ace, ade, bcd, bce, bde, cde, abcd, abce, acde, bcde, abcde.

Vorzugsweise werden als Pflegesubstanzen pflanzliche Extrakte eingesetzt. Weiterhin besonders bevorzugt Pflegesubstanzen sind Pflegesubstanzen, die keine deodorierende Wirkung zeigen.

Es ist erfindungsgemäß weiterhin bevorzugt, dass die Pflegesubstanz oder mindestens eine Substanz der Mischung der Pflegesubstanzen als funktionelle Gruppe eine Doppelbindung, eine OH-Gruppe, eine NH-Gruppe oder eine COOH-Gruppe oder ein Salz mindestens einer dieser Gruppen, vorzugsweise eine OH-Gruppe aufweisen.

Besonders bevorzugte Pflegesubstanzen sind ausgewählt aus der Gruppe bestehend aus 18-β-Glycyrrhetinsäure aus Süßholzwurzel-Extrakt (Gylcyrrhiza glabra), vorzugsweise in einer Reinheit von >99 % Reinsubstanz im Extrakt, Aescin in Rosskastanie (Aesculus hippocastanum), Allantoin, Aloe vera (beinhaltend hauptsächlich Zucker, Anthraquinone und Mineralien wie Zink), Aminosäuren wie beispielsweise Alanin, Arginin, Serin, Lysin, Ammoniumglycyrrhizat aus Süßholzwurzel-Extrakt, vorzugsweise in einer Reinheit von fast 100 % Reinsubstanz im Extrakt, Apigenin aus Kamille-Extrakt (Matricaria recutita), Arnica, insbesondere Arnica montana oder Arnica chamissonis, Asiaticoside und Madecassoside im Centella asiatica-Extrakt, Avenaanthramide aus Hafer-Extrakt (Avena sativa), Avocadol, Azulen aus Kamille-Extrakt (Matricaria recutita), Biotin (Vitamin H), Bisabolol aus Kamille-Extrakt (Matricaria recutita), Braunalgen-Extrakt (Ascophyllum nodosum), Chlorogensäure in Wasserextrakt des Japanischen Geißblatts (Lonicera japonica), Coenzym Q10, Creatin, Dexpanthenol, Dinatriumglycyrrhizat aus Süßholzwurzel-Extrakt, vorzugsweise in einer Reinheit von fast 100 % Reinsubstanz im Extrakt, Extrakt aus der Rotalge (Asparagopsis armata), Flavonoide aus Birken-Extrakt (Betula alba), Flavonoide, Vitexin im Extrakt der Passionsblume (Passiflora incarnata), Flavonoide, Vitexin im Linden-Extract (Tilia platyphyllos), Ginkgoflavonglykoside und Terpene Lactones im Ginkgo-Extract (Ginkgo biloba), Ginsenoside im Ginseng-Extrakt (Panax ginseng), Glykogen, Grapefruit-Extrakt, Hamamelis-Extrakt aus virginischer Zaubernuß (Hamamelis virgiana), Honig, Isoflavonglykoside im Klee-Extrakt (Trifolium pratense), Johanneskraut-Extrakt aus Johanneskraut (Hypericum perforatum), Jojobaöl, Lecithin, Maisöl (Zea mays), Nachtkerzenöl, Niacinamid, Oenotheine B im Extrakt aus Weidenröschen (Epilobium angustifolium), Oleuropein im Oliven-Extract (Olea europea), Phytocohesin (Natrium-Beta-Sitosterolsulfat), Plankton-Extrakt (Tetraselmis suecica, Spirulina und andere), Polyphenole, Catechine aus dem Extrakt von Traubenkernen (Vitis vinifera), Polyphenole, Catechine aus grünem Tee (Camellia sinensis), Ringelblumen-Extrakt (Calendula officinalis), Rosmarinsäure in Melissen-Extract (Melissa officinalis), Sandornöl, β-Glukane aus Hafer (Avena sativa), Stearylglycyrrhetinsäure (Stearylester der 18-β-Beta Glycyrrhetinsäure), Sterole, Sitosterol im Brennessel-Extrakt (Urtica dioca), Süßmandelöl (Prunus dulcis), Vitamin C und seine Ester, Vitamin E und seine Ester, Weizenkeimöl Zinkglukonat/Magnesiumaspartat/Kupferglukonat, sowie Zinksulfat oder Zinkoxid.

In einer bevorzugten Ausführungsform der erfindungsgemäßen wirkstoffdotierten wasserabsorbierenden Polymerteilchen beinhalten diese als Pflegesubstanz eine Mischung aus mindestens zwei der vorstehend genannten Pflegesubstanzen. In einer anderen bevorzugten Ausführungsform der erfindungsgemäßen wirkstoffdotierten wasserabsorbierenden Polymerteilchen beinhalten diese als Pflegesubstanz eine Mischung aus mindestens drei der vorstehend genannten Pflegesubstanzen.

Als Wundheilsubstanz werden vorzugsweise diejenigen Arzneimittelwirkstoffe mit wundheilender Wirkung verstanden, die unter den Arzneimittelbegriff des § 2 Arzneimittelgesetz in der Fassung vom 11. Dezember 1998 fallen, wobei die in der Roten Liste des Jahres 2002 angegebenen, wundeheilenden Wirkstoffe besonders bevorzugt sind.

Bei einer "Wundheilsubstanz" im Sinne dieser Erfindung handelt es sich um eine Substanz oder eine Mischung aus mindestens zwei Substanzen, die als solche oder in Kombination mit weiteren aktiven Komponenten in der Lage sind, den Prozess der Wundheilung zu fördern, sei es beispielsweise durch eine Desinfektionswirkung im Wundbereich, durch eine Förderung der Homeostase des Wundmilieus, durch eine Stimulation des Zellwachstums im Wundbereich oder durch eine Stimulation der Sekretion von beispielsweise Proteinen wie Kollagen oder Elastin, von Proteoglukanen oder von Botenstoffen durch die Hautzellen im Wundbereich.

Vorzugsweise weisen die erfindungsgemäßen "Wundheilsubstanzen" als funktionelle Gruppe eine Doppelbindung, eine OH-Gruppe, eine NH-Gruppe oder eine COOH-Gruppe oder ein Salz mindestens einer dieser Gruppen, vorzugsweise eine OH-Gruppe auf Zudem ist es bevorzugt, dass die Wundheilsubstanz eine 2 bis 100 Kohlenstoffatome und ein bis 20 Sauerstoffatome aufweist. Die vorstehenden Eigenschaften sind ebenfalls für die erfindungsgemäßen Wirkstoffe oder Arzneimittelwirkstoffe bevorzugt.

Im allgemeinen werden als auf Pflanzenextrakten basierende Wundheilsubstanzen Equisetum arvense, Aloe barbadensis, Arnica montana, Arnica chamissonis, Symphytum officinale, Solanum dulcamara, Echinacea pallida, Potentilla erecta, Trigonella foenum-graecum, Juglans regia, Linum usitatissimum, Terminalia sericea, Oenothera biennis, Centella asiatica, Arctium lappa, Capsella bursa-pastoris, Hypericum perforatum, Matricaria recutita, Chamomille recutita, Agrimonia eupatoria, Centaurea cyanus, Larrea tridentata, Populus spec., Echinacea pupurea, Calendula officinalis, Aesculus hippocastanum, Salvia officinalis, Plantago lanceolata, Quercus robur, Glycyrhiza glabra, Quercus petraea, Hamamelis virgian, Cardiospermum halicacabum,Betula, Urtica dioica, Buxus chinensis, Lavandula angustifolia, Lavandula hybrida, Crocus sativus, Smilax aspera, Melaleuca alternifolia, Aminosäuren oder Viola tricolor oder deren Salze oder Derivate oder Mischungen aus mindestens zwei davon erfindungsgemäß eingesetzt.

Ferner kommen als weitere Wundheilsubstanzen Vitamine und der gleichen wie Glukosamin Sulfate Allantoin, Biotin, Chondroitin Sulfate, Coenzym Q10, Dexpanthenol, Honig/Honigextrakt, Niacinamid, Propolis, Vitamin A oder seine Ester, Vitamin C und seine Ester, Vitamin E und seine Ester oder deren Salze oder Derivate oder Mischungen aus mindestens zwei davon erfindungsgemäß in Betracht.

Erfindungsgemäß bevorzugt handelt es sich bei Wundheilsubstanzen um Dexpanthenol oder Extrakte der Ringelblume, vorzugsweise Calendulaöl; der Hamamelis, vorzugsweise D-Hamelose; oder der Kamille, vorzugsweise das Öl der Kamillenblüte - vorzugsweise Bisbolol oder Azulen - oder Mischungen aus mindestens zwei der vorstehenden Substanzen.

Weiterhin ist es bevorzugt, dass jeweils eine der vorstehenden Wundheilsubstanzen in einer Mischung als Hauptkomponente in einer bevorzugten Ausführungsform dieser Erfindung vorliegen kann, wobei diese Hauptkomponente vorzugsweise mindestens 50 Gew.-%, vorzugsweise mindestens 70 Gew.-% und besonders bevorzugt mindestens 95 Gew.-%, jeweils bezogen auf die Mischung, vorliegen kann.

Wie ein Vergleich der Liste der bevorzugten Pflegesubstanzen mit der Liste der bevorzugten Wundheilsubstanzen zeigt, können als Wirkstoffe auch Verbindungen oder Zusammensetzungen eingesetzt werden, die sowohl eine Wundheilende als auch eine pflegende Wirkung haben, wobei derartige Wirkstoffe erfindungsgemäß besonders bevorzugt sind. Zu diesen Wirkstoffen gehören beispielsweise Allantonin, recutita), Arnica, insbesondere Arnica montana oder Arnica chamissonis, Biotin, Coenzym Q10, Dexpanthenol, Honig oder Honigextrakt, Aminosäure, Niacinamid, Vitamin C oder seine Ester oder Vitamin E oder seine Ester.

Es ist weiterhin bevorzugt, dass als Wirkstoff eine Mischung aus einer wundheilenden und einer pflegenden Subtanz eingesetzt wird. Außerdem umfassen die erfindungsgemäß angegebenen Wirkstoffe auch deren entsprechend wirksamen Salze.

Die wirkstoffdotierten wasserabsorbierenden Polymerteilchen werden vorzugsweise in Hygieneartikeln, beispielsweise in Windeln oder Damenbinden, eingesetzt.

Zur Lösung der vorstehenden Aufgaben betrifft die Erfindung ferner eine wasserabsorbierende Zusammensetzung, beinhaltend
Γ1. eine Polykondensatmatrix, basierend auf mindestens einem Polykondensatmonomer mit mindestens einer Polykondensatgruppe, und
Γ2. ein erfindungsgemäßes wirkstoffdotiertes wasserabsorbierendes Polymerteilchen,
wobei das teilchenförmige wasserabsorbierende Polymer oder das wirkstoffdotierte wasserabsorbierende Polymerteilchen oder beide mindestens teilweise von der Polykondensatmatrix umgeben ist,
wobei mindestens das teilchenförmige wasserabsorbierende Polymer oder das wirkstoffdotierte wasserabsorbierende Polymerteilchen oder beide die Wundheilsubstanz oder den Wirkstoff oder beide zu mindestens 60, vorzugsweise mindestens 80 und besonders bevorzugt mindestens 90 Gew.-%, jeweils bezogen auf die in der Zusammensetzung enthaltende Menge, aufweisen, und vorzugsweise
wobei die wasserabsorbierende Zusammensetzung eine Wirkstoffverfügbarkeit von mindestens 10 Gew.-%, vorzugsweise mindestens 30 Gew.-%, weiterhin bevorzugt mindesten 70 Gew.-%, ferner bevorzugt mindestens 80 Gew.-% und darüber hinaus bevorzugt mindestens 90 Gew.-% des in der wasserabsorbierenden Zusammensetzung enthaltenen Wirkstoffes nach dem hierin angegebenen Extraktions-Test zeigt.

In einer weiteren bevorzugten Ausführungsform der erfindungsgemäßen Zusammensetzung weist diese in Γ2. ein teilchenförmiges wasserabsorbierendes Polymer, beinhaltend einen Wirkstoff, vorzugsweise eine Wundheilsubstanz oder eine Hautpflegesubstanz, oder deren Salz, mit mindestens einer funktionellen Gruppe, die mit der Polykondensatgruppe unter Bildung einer kovalenten Bindung reagieren kann, auf.

Unter "wasserabsorbierend" wird erfindungsgemäß neben der Fähigkeit eines Stoffes, Wasser - vorzugsweise mindestens das vierfache, besonders bevorzugt mindestens das 10-fache und besonders bevorzugt mindestens das 100-fache seines Eigengewichts - in sich unter Bildung eines Hydrogels aufzunehmen, jegliche Aufnahme von wässrigen Flüssigkeiten, insbesondere wässrigen Körperflüssigkeiten wie Urin, Blut, Blutbestandteile wie Eiter, Lymphflüssigkeiten oder Blutserum, verstanden.

Als Wundheilsubstanzen und Pflegesubstanzen kommen vorzugsweise diejenigen Wundheilsubstanzen und Pflegesubstanzen oder Mischungen aus Wundheilsubstanzen und Pflegesubstanzen in Betracht, die im Zusammenhang mit den erfindungsgemäßen wirkstoffdotierten wasserabsorbierenden Polymerteilchen als bevorzugte Wundheilsubstanzen und Pflegesubstanzen genannt wurden.

Gemäß einer Ausführungsform der erfindungsgemäßen wasserabsorbierende Zusammensetzung, beinhaltet diese als Zusammensetzungskomponenten
a. eine Polykondensatmatrix, in einer Menge im Bereich von 5 bis 98,999 Gew.-%, vorzugsweise im Bereich von 20 bis 95,9 Gew.-% und besonders bevorzugt im Bereich von 30 bis 93,9 Gew.-%, jeweils bezogen auf die wasserabsorbierende Zusammensetzung;
b. ein teilchenförmiges wasserabsorbierendes Polymer, in einer Menge im Bereich von 1 bis 70 Gew.-%, vorzugsweise im Bereich von 3 bis 25 Gew.-% und besonders bevorzugt im Bereich von 5 bis 15 Gew.-%, jeweils bezogen auf die wasserabsorbierende Zusammensetzung;
c. einen Wirkstoff, vorzugsweise eine Wundheilsubstanz oder eine Hautpflegesubstanz, beinhaltet in dem teilchenförmigen wasserabsorbierenden Polymer, in einer Menge im Bereich von 0,001 bis 25 Gew.-%, vorzugsweise im Bereich von 0,01 bis 20 Gew.-% und besonders bevorzugt im Bereich von 0,1 bis 15 Gew.-%, jeweils bezogen auf die wasserabsorbierende Zusammensetzung; sowie
d. ein oder mehrere Additive, in einer Menge im Bereich von 0 bis 50 Gew.-%, vorzugsweise im Bereich von 0,1 bis 40 Gew.-% und besonders bevorzug im Bereich von 1 bis 30 Gew.-%, jeweils bezogen auf die wasserabsorbierende Zusammensetzung;
wobei die Summe der Gewichtsmengen der Zusammensetzungskomponenten a. bis d. 100 Gew.-% ergibt. Die Zusammensetzung dieser Ausführungsform wird vorzugsweise in Wundauflagen und Hauptpflastern, vorzugsweise in Wundpflastern eingesetzt.

Die nachfolgenden Konzentrations- und Gewichtsanteilangaben für Hautpflegemittel gelten in Wirkstoffe betreffenden Ausführungsformen dieser Erfindung ebenso.

Weiterhin betrifft die Erfindung ein Verfahren zu Herstellung einer wasserabsorbierenden Zusammensetzung, wobei ein einen Wirkstoff, vorzugsweise eine Wundheilsubstanz oder eine Hautpflegesubstanz beinhaltendes teilchenförmiges wasserabsorbierendes Polymer mindestens teilweise in eine auf mindesten einem Polykondensatmonomer basierenden Polykondensatmatrix eingearbeitet wird, wobei ein erfindungsgemäßes wirkstoffdotiertes wasserabsorbierendes Polymerteilchen mit dem Polykondensatmonomer vor Abschluss der Polykondensatmatrixbildung in Kontakt gebracht wird.

In dem erfindungsgemäßen Verfahren ist es bevorzugt, dass der Wirkstoff in das den Wirkstoff beinhaltende wasserabsorbierenden Polymer vor Abschluss der Bildung des wasserabsorbierenden Polymers eingearbeitet wurde. Erfindungsgemäß wird von dem Abschluss der Bildung des wasserabsorbierenden Polymers vorzugsweise dann ausgegangen, wenn der Gehalt an restlichem Monomeren, auf dem das wasserabsorbierende Polymer basiert, unter 500 ppm, vorzugsweise unter 300 ppm, ferner bevorzugt unter 200 ppm und darüber hinaus bevorzugt unter 146 ppm, jeweils bezogen auf das wasserabsorbierende Polymer, liegt. Der Restmonomergehalt wird nach ERT 410.1-99 bestimmt.

Der geringe Restmonomergehalt wirkt sich wundheilfördernd und hautpflegend aus und trägt zur Verbesserung des Tragekomforts von Hautpflastern, insbesondere Wundpflastern oder Wundauflagen bei, da Gewebe- und insbesondere Hautirritationen abgeschwächt oder gar vermieden werden, die bei längeren Tragen des Hautpflasters oder der Wundauflage leicht entstehen können.

Es ist weiterhin bevorzugt, dass der Wirkstoff bereits zu den Monomeren vor Beginn der Polymerisationsreaktion zur Bildung des wasserabsorbierenden Polymers hinzugegeben wird, so dass der Wirkstoff während der Polymerisationsreaktion in das wasserabsorbierende Polymer eingearbeitet wird.

Die Einarbeitung des Wirkstoffes in das wasserabsorbierende Polymer während der Polymerisation des wasserabsorbierenden Polymers kann durch alle dem Fachmann geläufigen Verfahren erfolgen. Zum einen kann der Wirkstoff über das zur Herstellung des wasserabsorbierenden Polymers verwendeten Lösemittel in das wasserabsorbierende Polymer eingearbeitet werden. Zum anderen kann der Wirkstoff dem zur Bildung des wasserabsorbierenden Polymers verwendeten Monomer, Oligomer oder Präpolymer oder mindestens zwei davon zugesetzt werden. In beiden vorstehenden Varianten kann der Wirkstoff als Lösung, Emulsion oder Suspension vorliegen. Weiterhin können die beiden vorstehenden Varianten miteinander kombiniert werden.

In einer anderen Ausführungsform des erfindungsgemäßen Verfahren wird der Wirkstoff nach Abschluss der Bildung des wasserabsorbierenden Polymers oder während dessen Weiterverarbeitung oder beides eingearbeitet. Diese Einarbeitung erfolgt vorzugsweise in ein Gel des wasserabsorbierenden Polymers. Hierbei ist es bevorzugt, dass das Gel eine auf das wasserabsorbierenden Polymer bezogene Wassermenge vom 0,2- bis 20-fachen, vorzugsweise 1- bis 10-fachen und besonders bevorzugt 2- bis 4-fachen aufweist, um eine möglichst gleichmäßige Einarbeitung des Wirkstoffes zu erreichen.

Dieses kann zum einen durch Absorption des Wirkstoffes mittels eines flüssigen, meist wässrigen Trägers erfolgen, in dem der Wirkstoff vorzugsweise gelöst ist. Bei der Einarbeitung des Wirkstoffes im Zuge der Weiterverarbeitung ist es bevorzugt, dass der Wirkstoff in einer flüssigen, vorzugsweise wässrigen Phase in das wasserabsorbierende Polymer, ggf. während der nachfolgend beschriebenen "Nachvernetzung", mit der eine Oberflächen Vernetzung erreicht wird, eingearbeitet wird. Dieses erfolgt vorzugsweise zusammen mit den zu den hierzu verwendeten Nachvernetzern.

Es sind auch Kombinationen der vorstehenden Verfahrensvarianten möglich. Bei der Einarbeitung des Wirkstoffes vor Abschluss der Bildung des wasserabsorbierenden Polymers wird vorzugsweise eine gleichmäßige Dotierung des wasserabsorbierenden Polymers erreicht. Wird der Wirkstoff nach Abschluss der Bildung des wasserabsorbierenden Polymers oder während dessen Weiterverarbeitung oder beides eingearbeitet, so wird vorzugsweise eine Dotierung des wasserabsorbierenden Polymerteilchens in dessen Außen- oder Oberflächenbereich erhalten.

Eine Kombination der beiden Verfahrensvarianten führt in aller Regel zu einem Polymer mit einer unterschiedlichen Konzentration im Innen- und Außenbereich des wasserabsorbierenden Polymerteilchens, wobei die Konzentration an Wirkstoff im Außenbereich meist höher ist.

Das so erhaltene mit Wirkstoff dotierte wasserabsorbierende Polymer, nachfolgend "dotiertes Polymer" genannt, kann allgemein auf jede dem Fachmann bekannte weise in die Polykondensatmatrix eingearbeitet werden. Es ist bevorzugt, dass das dotierte Polymer vor Abschluss der Bildung, d. h. bevor im wesentliche alle reaktiven funktionellen Gruppen eines Polykondensatmatrixmonomers abreagiert sind, in die Polykondensatmatrix eingearbeitet werden. Dieses erfolgt vorzugsweise dadurch, dass das dotierte Polymer entweder dem zur Bildung der Polykondensatmatrix verwendeten Lösemittel oder einem Polykondensatmatrixmonomer beigegeben wird. Vorzugsweise erfolgt die Beigabe zu einem Polykondensatmatrixmonomer, wobei die Zugabe zu einem Polykondensatmatrixmonomer, das frei ist von reaktiven funktionellen Gruppen, vorzugsweise frei von reaktiven funktionellen Gruppen, die mit funktionellen Gruppen des dotierten Polymers reagieren können, beispielsweise einem Polyol im Fall der als Polykondensatmatrix bevorzugten Polyurethanmatrix, besonders bevorzugt ist.

Die Erfindung betrifft auch ein Verfahren zur Herstellung eines wirkstoffdotierten wasserabsorbierenden Polymerteilchens, wobei eine Pflegesubstanz oder eine Wundheilsubstanz in eine Absorbermatrix eingearbeitet wird,
wobei die Absorbermatrix zu mindestens 90 Gew.-%, bezogen auf die Absorbermatrix, auf einer vernetzten Polyacrylsäure besteht, und
wobei die vernetzte Polyacrylsäure zu mindestens 90 Gew.-%, bezogen auf die vernetzte Polyacrylsäure, aus einer zu mindestens 30 Mol-% teilneutralisierten Acrylsäure besteht.

Als wasserabsorbierendes Polymer sind dabei diejenigen Polymere bevorzugt, die bereits im Zusammenhang mit dem erfindungsgemäßen Verfahren zur Herstellung der wasserabsorbierenden Zusammensetzung genannt wurden. Bevorzugte Pflegemittel und Wundheilsubstanzen sind diejenigen, die im Zusammenhang mit den erfindungsgemäßen wirkstoffdotierten Polymerteilchen genannt wurden.

Das Einarbeiten der Pflegesubstanz oder der Wundheilsubstanz in die Absorbermatrix kann dabei, wie vorstehend im Zusammenhang mit dem Einarbeiten des Wirkstoffes in das wasserabsorbierende Polymer beim erfindungsgemäßen Verfahren zur Herstellung einer wasserabsorbierenden Zusammensetzung beschrieben, während der Polymerisationsreaktion, also vor Abschluss der Bildung des wasserabsorbierenden Polymerteilchens, oder nach Abschluss der Bildung des wasserabsorbierenden Polymers oder während dessen Weiterverarbeitung oder beides eingearbeitet werden. Hinsichtlich der Art und Weise der Verfahrensführungen wird auf die Ausführungen im Zusammenhang mit dem Einarbeiten des Wirkstoffes in das wasserabsorbierende Polymer beim erfindungsgemäßen Verfahren zur Herstellung der wasserabsorbierenden Zusammensetzung verwiesen.

Es ist erfindungsgemäß bevorzugt, dass die Pflegesubstanz oder die Wundheilsubstanz in einer solchen Menge in die Absorbermatrix eingearbeitet wird, dass das entstehende wirkstoffdotierte wasserabsorbierende Polymerteilchen auf
Λ1. 0,001 bis 30 Gew.-%, vorzugsweise von 0,1 bis 20 Gew.-% und besonders bevorzugt von 1 bis 15 Gew.-% Pflegesubstanz oder Wundheilsubstanz und
A2. 80 bis 99,9 Gew.-% und besonders bevorzugt von 90 bis 99 Gew.-% Absorbermatrix basiert, wobei die Summe der Komponenten Λ1 und Λ2. 100 Gew.-% beträgt.

Zudem betrifft die Erfindung eine wasserabsorbierende Zusammensetzung und wirkstoffdotierte wasserabsorbierende Polymerteilchen, erhältlich nach den vorstehenden Verfahren.

Bei der erfindungsgemäßen Zusammensetzung und den wirkstoffdotierten wasserabsorbierenden Polymerteilchen ist es bevorzugt, dass das wasserabsorbierende Polymer mindestens eine, vorzugsweise jede, der nachfolgenden Eigenschaften aufweist:
A1) eine Partikelgrößenverteilung aufweisen, wobei mindestens 80 Gew.-% der Partikel eine Partikelgröße in einem Bereich von 20 µm bis 900 µm, vorzugsweise im Bereich von 150 µm bis 600 µm und besonders bevorzugt im Bereich von 200 µm bis 400µm nach ERT 420.1-99 besitzen;
A2) eine Centrifuge Retention Capacity (CRC) von mindestens 10 g/g, vorzugsweise mindestens 20 g/g und besonders bevorzugt in einem Bereich von 30 bis 50 g/g nach ERT 441.1-99;
A3) eine Absorption Against Pressure (AAP) bei 0,7 psi (4826 Pa) von mindestens 4 g/g, vorzugsweise mindestens 6 g/g und besonders bevorzugt in einem Bereich von 8 bis 25 g/g nach ERT 442-1.99;
A4) einen Gehalt an wasserlöslichem Polymer nach 16 Stunden Extraktion von weniger als 25 Gew.-%, vorzugsweise weniger als 20 Gew.-% und besonders bevorzugt weniger als 15 Gew.-%, jeweils bezogen auf das Gesamtgewicht des wasserabsorbierenden Polymers, nach ERT 470.1-99,
A5) eine Restfeuchtigkeit von höchstens 15 Gew.-%, vorzugsweise höchstens 10 Gew.-% und besonders bevorzugt höchstens 5 Gew.-%, jeweils bezogen auf das Gesamtgewicht des wasserabsorbierenden Polymers, nach ERT 430.1-99.

Jeder der aus den Merkmalen A1 bis A5 ergebenden Merkmalskombinationen stellen eine erfindungsgemäße bevorzugte Ausführungsform dar, wobei die nachstehenden Merkmalskombinationen jeweils für sich besonders bevorzugte Ausführungsformen darstellen: A1A2, A1A2A3, A1A2A3A4, A1A3, A1A4, A1A3A4, A1A2A4, A2A3, A2A3A4, A2A4 sowie A3A4, wobei alle der vorstehenden Kombinationen mit A1 besonders bevorzugt sind.

Bei einer werters offenborten wasserabsorbierenden Zusammensetzung und den wirkstoffdotierten wasserabsorbierenden Polymerteilchen ist es weiterhin bevorzugt, dass das wasserabsorbierende Polymer auf
(α1) 0,1 bis 99,999 Gew.-%, bevorzugt 20 bis 98,99 Gew.-% und besonders bevorzugt 30 bis 98,95 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure, beinhaltende Mischungen besonders bevorzugt sind,
(α2) 0 bis 70 Gew.-%, bevorzugt 1 bis 60 Gew.-% und besonders bevorzugt 1 bis 40 Gew.-% polymerisierten, ethylenisch ungesättigten, mit (α1) copolymerisierbaren Monomeren,
(α3) 0,001 bis 10 Gew.-%, bevorzugt 0,01 bis 7 Gew.-% und besonders bevorzugt 0,05 bis 5 Gew.-% eines oder mehrerer Vernetzer,
(α4) 0 bis 30 Gew.-%, bevorzugt 1 bis 20 Gew.-% und besonders bevorzugt 5 bis 10 Gew.-% wasserlöslichen Polymeren, sowie
(α5) 0 bis 20 Gew.-%, bevorzugt 0,01 bis 7 Gew.-% und besonders bevorzugt 0,05 bis 5 Gew.-% eines oder mehrere Hilfsstoffe basiert,
wobei die Summe der Gewichtsmengen (α1) bis (α5) 100 Gew.-% beträgt.

Die monoethylenisch ungesättigten, säuregruppenhaltigen Monomere (α1) können teilweise oder vollständig, bevorzugt teilweise neutralisiert sein. Vorzugsweise sind die monoethylenisch ungesättigten, säuregruppenhaltigen Monomere zu mindestens 25 Mol-%, besonders bevorzugt zu mindestens 50 Mol-% und darüber hinaus bevorzugt zu 50-90 Mol-% neutralisiert. Die Neutralisation der Monomere (α1) kann vor und auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Bevorzugte monoethylenisch ungesättigte, säuregruppenhaltige Monomere (α1) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere (α1) des Weiteren ethylenisch ungesättigte Sulfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat und, 2-Hydroxy-3-methacryloxypropylsulfonsäure. Als (Meth)Acrylamidoalkylsulfonsäure ist 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acrylphosphonsäurederivate bevorzugt.

Die erfindungsgemäße Absorber matrix besteht zu mindestens 90 Gew.-%, bezogen auf die Absorber matrix, aus einer vernetzten Polyacrylsäure, wobei die vernetzte Polyacrylsaure zu mindestens 90 Gew.-%, bezogen auf die vernetzte Polyacrylsäure, aus einer zu mindestens 30 Mol-% teilneutralisierten Acrylsäure besteht.

Als ethylenisch ungesättigte, einen protonierten Stickstoff enthaltende Monomere (α1) sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydrosulfat, sowie Dialkylaminoalkyl-(meth)acrylamide in protonierter Form, beispielsweise Dimethylaminoethyl(meth)-acrylamid-Hydrochlorid, Diemethylaminopropyl(meth)acrylamid-Hydrochlorid, Dimethylaminopropyl(meth)acrylamid-Hydrosulfat oder Dimethylaminoethyl(meth)-acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamidoalkyldialkylamine in quarternisierter Form, beispielsweise (Meth)acrylamidopropyl-trimethylammoniumchlorid, Tremethylammoniumethyl(meth)acrylat-Chlorid oder (Meth)acrylamidopropyltrimethylammoniumsulfat bevorzugt.

Als monoethylenisch ungesättigte, mit (α1) copolymerisierbare Monomere (α2) sind Acrylamide und Methacrylamide bevorzugt.

Mögliche (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2). Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'(1,2-Di-hydroxyethylen)bis(meth)acrylamid, N,N'-Hexamethylenbis(meth)acrylamid oder N,N'-Methylenbis(meth)acrylamid, Polyalkoxydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acrylat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammoniumchlorid und Homo- und Copolymere von Diethyl(meth)allylaminomethyl(meth)acrylatammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri(meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri(meth)allylphosphat Tetra(meth)allylethylendiamin, Poly(meth)allylester, Tetra(meth)allyloxiethan oder Tetra(meth)allylammoniumhalide. Erfindungsgemäß sind in der Vernetzerklasse I Vinylisocyanate, Trivinyltrimellitat oder Tri(meth)allylisocyanurat bevorzugt, wobei Trivinyltrimellitat besonders bevorzugt sind.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere (α1) oder (α2), bevorzugt mit Säuregruppen, der Monomeren (α1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonatoder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat, sowie hydroxyl- oder aminogruppenhaltige (Meth)acrylamide, oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O NaAl(SO₄)₂ × 12 H₂O KAl(SO₄)₂ × 12 H₂O oder Al₂(SO₄)₃×14-18 H₂O eingesetzt.

Besonders bevorzugt werden Al₂(SO₄)₃ und seine Hydrate als Vernetzer der Vernetzungsldasse IV verwendet.

Bevorzugte sind wasserabsorbierende Polymere, die durch Vernetzer der folgenden Vernetzerklassen bzw. durch Vernetzer der folgenden Kombinationen von Vernetzerklassen vernetzt sind: I, II, III, IV, I II, I III, I IV, I II III, I II IV, I III IV, II III IV, II IV oder III IV. Die vorstehenden Kombinationen von Vernetzerklassen stellen jeweils für sich eine bevorzugte Ausführungsform von Vernetzern eines wasserabsorbierenden Polymers dar.

Einer weiteren bevorzugten Ausführungsform entsprechen wasserabsorbierende Polymere, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den erfindungsgemäßen wasserabsorbierenden Polymeren wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange diese wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (α5) werden vorzugsweise Stellmittel, Geruchsbinder, oberflächenaktive Mittel oder Antioxidatien eingesetzt.

Aus den vorgenannten Monomeren und Vernetzern lässt sich das wasserabsorbierende Polymer durch verschiedene Polymerisationsweisen herstellen. Beispielsweise sind in diesem Zusammenhang Massepolymerisation, die vorzugsweise in Knetreaktoren wie Extrudern oder durch Bandpolymerisation erfolgt, Lösungspolymerisation, Spraypolymerisation, inverse Emulsionspolymerisation und inverse Suspensionspolymerisation zu nennen. Bevorzugt wird die Lösungspolymerisation in Wasser als Lösungsmittel durchgeführt. Die Lösungspolymerisation kann wie die anderen vorstehend genannten Polymerisationsarten kontinuierlich oder diskontinuierlich erfolgen. Vorzugsweise erfolgt die Lösungspolymerisation als kontinuierliche laufende Bandpolymerisation. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818. Im Falle der vorstehend genannten Polymerisationsarten ist es bevorzugt, die Wundheilsubstanz bereits mit dem oder den in dem entsprechenden Polymerisationsverfahren eingesetzten Lösemittel oder Monomer bzw. Monomeren in den zuvor bereits beschriebenen Varianten als Gemisch in die Polymerisation einzuführen.

Eine andere Möglichkeit zur Herstellung der wasserabsorbierenden Polymere besteht darin, zunächst unvernetzte, insbesondere lineare Präpolymere, vorzugsweise auf radikalischem Wege aus den vorgenannten monoethylenisch ungesättigten Monomeren (α1) bzw. (α2) herzustellen und diese dann mit vernetzend wirkenden Reagenzien (α3), vorzugsweise denen der Klassen II und IV, umzusetzen. Diese Variante wird vorzugsweise dann eingesetzt, wenn die wasserabsorbierenden Polymeren zunächst in formgebenden Verfahren, beispielsweise zu Fasern, Folien oder anderen Flächengebilden; wie Geweben, Gewirken, Gespinsten oder Vliesen verarbeitet und in dieser Form vernetzt werden sollen. Im Zusammenhang mit diesen Herstellungsverfahren kann ein Wirkstoff bzw. eine Pflegesubstanz zum einen bei der Herstellung des Präpolymeren eingearbeitet werden. Anderseits kann der Wirkstoff bzw. die Pflegesubstanz bei der Vernetzung der Präpolymere eingearbeitet werden.

Bei der Herstellung der wasserabsorbierenden Polymere werden die dem Fachmann zur Herstellung solcher Polymere bekannten Lösungsmittel, vorzugsweise Wasser, die üblichen Temperaturbereiche, vorzugsweise bei einer Temperatur von 1 bis 100°C, besonders bevorzugt 3 bis 60°C, und Drücke im Normaldruckbereich eingehalten.

Das durch vorstehende Verfahren erhältliche wasserabsorbierende Polymer kann, beispielsweise wenn ein festeres Gel gewünscht wird, einer weiteren Vernetzungsreaktion, in der ein Teil der Säuregruppen dieses Polymers vorzugsweise im Bereich der Oberfläche durch zumindest bifunktionelle Verbindungen vernetzt werden, unterworfen werden. Diese Reaktion wird allgemein als "Nachvernetzung" bezeichnet. Zu Einzelheiten der Nachvernetzung wir neben dem Nachfolgenden auf DE 40 20 780 C1 verwiesen. Die Nachvernetzung bietet sich beispielsweise dann an, wenn die erfindungsgemäßen wirkstoffdotierten wasserabsorbierenden Polymerteilchen oder die erfindungsgemäße Zusammensetzung bei der Wundheilung erhöhtem Druck ausgesetzt sind, wie es beispielsweise bei Pressverbänden der Fall sein kann.

Bevorzugte Ausführungsformen der Polymere sind diejenigen, die durch Vernetzer der folgenden Vernetzerklassen bzw. durch Vernetzer der folgenden Kombinationen von Vernetzerklassen nachvernetzt sind: II, IV und II IV. Als Nachvernetzer besonders bevorzugt sind die im Zusammenhang mit den Vernetzern genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Weitere bevorzugte Ausführungsformen der Polymere sind diejenigen, die durch einen beliebigen der vorstehend in den Vernetzerklassen II oder IV genannten Vernetzer nachvernetzt sind.

Diese Verbindungen werden vorzugsweise in einer Menge im Bereich von 0,01 bis 30, bevorzugt 0,1 bis 20 und besonders bevorzugt 0,5 bis 10 Gew.%, bezogen auf das noch unbehandelte Polymer eingesetzt. Organische Lösungsmittel können der Mischung in einer Menge von 0 bis 60, bevorzugt 0,1 bis 40 und besonders bevorzugt 0,2 bis 50 Gew.%, bezogen auf das noch unbehandelte Polymer, zugesetzt werden. Als organische Lösungsmittel sind bevorzugt niedere Alkohole wie Methanol, Ethanol, n-Propanol, Isopropanol, n-Butanol, Isobutanol, sec-Butanol und t-Butanol, Ketone wie Aceton, Methylethylketon und Methylisobutylketon, Ether wie Dioxan, Tetrahydrofuran und Diethylether, Amide wie N,N-Dimethylformamid und N,N-Diethylformamid sowie Sulfoxide wie Dimethylsul-foxid.

Zudem ist eine erfindungsgemäße wasserabsorbierende Zusammensetzung bevorzugt, bei der die Polykondensatmatrix zu mindestens 10 Gew.-%, bevorzugt zu mindestens 50 Gew.-% und besonders bevorzugt zu mindestens 80 Gew.-%, jeweils bezogen auf die Polykondensatmatrix, ein Polyurethan aufweist. In diesem Zusammenhang kann der von Polyurethan verschiedene Teil ein anderes von den Zusammensetzungskomponenten verschiedenes Polymer sein, mit dem das Polyurethan beispielsweise durch Coextrusion vermischt ist. Vorzugsweise handelt es hierbei um ein weiteres wasserunlösliches, nicht wasserquellbares, thermoplasti-sches Polymer.

Als erfindungsgemäße Polyurethane kommen alle dem Fachmann geeignete Polyurethane in Betracht. Geeignete Polyurethane sind erhältlich mindestens aus den Polyurethankomponenten:
(γ1) einem oder mehren Polyisocyanaten mit mindestens zwei Isocyanatgruppen, vorzugsweise Methylendiphenyldiisocyanat (MDI), Toluylendiisocyanat (TDI) oder Isophorondiisocyant (IPDI) oder eine Mischung aus mindestens zwei davon;
(γ2) einer oder mehrer Polyhydroxylverbindungen, vorzugsweise mit einer mittleren OH-Zahl im Bereich von 20 bis 112;
(γ3) gegebenenfalls einen oder mehrere Katalysatoren oder Beschleuniger, jeweils für die Reaktion zwischen Isocyanat- und Hydroxylgruppen; sowie
(γ4) gegebenenfalls in der Polyurethanchemie bekannten Füll- und Zusatzstoffen

Weitere Einzelheiten zu den vorstehend genannten (γ1) bis (γ4) ergeben sich aus DE 42 33 289 A1 sowie DE 196 18 825 A1.

Vorzugsweise besitzen die erfindungsgemäße wasserabsorbierende Zusammensetzung und die erfindungsgemäßen wirkstoffdotierten wasserabsorbierenden Polymerteilchen mindestens einer, vorzugsweise jede, der folgenden Eigenschaften:
C1) einen Restmonomergehalt eines zur Bildung des wasserabsorbierenden Polymers eingesetzten Monomers von weniger als 500 ppm, vorzugsweise weniger als 250 ppm und besonders bevorzugt weniger als 146 ppm, jeweils bezogen auf das in der wasserabsorbierenden Zusammensetzung bzw. in den wirkstoffdotierten wasserabsorbierenden Polymerteilchen eingesetzte wasserabsorbierende Polymer, bestimmt nach ERT 410.1-99;
C2) einen Anteil löslicher Polymere nach 16 Stunden Extraktion, basierend auf den zur Bildung des wasserabsorbierenden Polymers eingesetzten Monomeren, von weniger als 30 Gew.-%, vorzugsweise weniger als 20 Gew.-%, besonders bevorzugt weniger als 15 Gew.-% und darüber hinaus bevorzugt weniger als 12 Gew.-%, jeweils bezogen auf das in der wasserabsorbierenden Zusammensetzung bzw. in den wirkstoffdotierten wasserabsorbierenden Polymerteilchen eingesetzte wasserabsorbierende Polymer, bestimmt nach ERT 470.1-99;
C3) eine Wirkstoff- bzw. Pflegesubstanzverfügbarkeit von mindestens 40 Gew.-%, vorzugsweise mindestens 70 Gew.-%, besonders bevorzugt mindestens 85 Gew.-% und darüber hinaus bevorzugt mindestens 95 Gew.-% eines in der wasserabsorbierenden Zusammensetzung bzw. in den wirk-stoffdotierten wasserabsorbierenden Polymerteilchen enthaltenen Wirkstoffs bzw. Pflegesubstanz, bestimmt nach dem hierin beschriebenen Extraktions-Test;
C4) eine Flüssigkeitsaufnahme von mindestens 1, vorzugsweise mindestens 4 und besonders bevorzugt mindestens 9 g / 100 cm²;
C5) eine Wasserdampfdurchlässigkeit von mindestens 100, vorzugsweise mindestens 200 und besonders bevorzugt mindestens 249 g / (m² x 24h); oder
C6) eine Sauerstoffdurchlässigkeit von mindestens 100, vorzugsweise mindestens 1000 und besonders bevorzugt mindestens 1999 cm³ / (m² x 24h).

Jede der sich aus den Merkmalen C1 bis C6 ergebenden Merkmalskombinationen stellt eine erfindungsgemäße bevorzugte Ausführungsform dar, wobei die nachstehenden Merkmalskombinationen jeweils für sich besonders bevorzugte Aus-führungsformen darstellen: C2C3C4C5C6, C1C3C4C5C6, C1C2C3C4C6, C1C2C4C5C6, C1C4C5C6, C2C4C5C6, C3C4C5C6, C1C4C5, C1C5C6, C3C5C6, C4C5C6, C3C4, C3C5, C3C6, C1C2, C2C3 oder C1C3.

Es ist weiterhin bevorzugt, dass in der erfindungsgemäßen wasserabsorbierenden Zusammensetzung die Polykondensatmatrix als Schaum vorliegt. Dieser wird durch dem Fachmann für diesen Zweck bekannte Treibmittel im Verlauf der Bildung des Polyurethans erzeugt. Hierzu wird auf die entsprechenden Ausführungen in DE 42 33 289 A1 zu Schäumungsmitteln und deren Verwendung verwiesen.

Ferner betrifft die Erfindung einen Verbund, beinhaltend eine erfindungsgemäße wasserabsorbierende Zusammensetzung oder die wirkstoffdotierten wasserabsorbierenden Polymerteilchen.

In einer Gestaltung des Verbundes ist es bevorzugt, dass dieser mindestens eine, vorzugsweise jede, der folgenden Eigenschaften zeigt:
V1) einer Viskoselastizität im Bereich von 0,1 bis 10, vorzugsweise im Bereich von 0,15 bis 7 und besonders bevorzugt im Bereich von 2 bis 5 tanδ (ω= 0,3 rad/s);
V2) einer Flüssigkeitsaufnahme von mindestens 5, mindestens 8 und besonders bevorzugt im Bereich von 10 bis 1000 sowie darüber hinaus bevorzugt im Bereich von 10,5 bis 20 g/100cm²;
V3) einer Wasserdampfdurchlässigkeit von mindestens 100, vorzugsweise mindestens 200 und besonders bevorzugt im Bereich von g/(m²x24h); oder
V4) einer O₂-Durchlässigkeit von mindestens 100, vorzugsweise mindestens 1000 und besonders bevorzugt im Bereich von 1100 bis 10000 sowie darüber hinaus bevorzugt im Bereich von 1500 bis 3000 cm³/(m²×24h).

Jede der sich aus den Merkmalen V1 bis V4 ergebenden Merkmalskombinationen stellt eine erfindungsgemäße bevorzugte Ausführungsform des Verbunds dar, wobei die nachstehenden Merkmalskombinationen jeweils für sich besonders bevorzugte Ausführungsformen darstellen: V1V2, V1V3, V1V4, V1V2V3, V1V2V4, V1V3V4, V2V3V4 oder V1V2V3V4.

In einer erfindungsgemäßen Ausführungsform beinhaltet der Verbund neben der erfindungsgemäßen Zusammensetzung oder den erfindungsgemäßen Polymerteilchen noch einen Film, welcher vorzugsweise unmittelbar an die Zusammensetzung oder die Polymerteilchen angrenzt. Besonders bevorzugt füllen die Zusammensetzung oder die Polymerteilchen in Form einer dünnen Schicht mindestens 40 % vorzugsweise mindestens 70 % und darüber hinaus bevorzugt mindestens 90 % der Fläche einer Seite des Films aus. Ferner ist es bevorzugt, dass der Film und die Zusammensetzung bzw. die Polymerteilchen fest mit einander verbunden sind. Dieses erfolgt vorzugsweise durch die Haftwirkung der Matrix der Zusammensetzung. Ferner ist es bevorzugt, dass der Film eine Wasserdampfdurchlässigkeit im Bereich von 100 bis 2000, vorzugsweise im Bereich von 300 bis 1500 und besonders im Bereich von 500 bis 1000 g/(m²×24h) besitzt. Als Film kommen alle dem Fachmann geläufigen und als geeignet bekannten Materialien in betracht. In diesem Zusammenhang sind Kunststofffilme, vorzugsweise hauptsächlich beinhaltend Polyethylen oder Polypropylen oder deren Mischungen, bevorzugt.

Wenn der Verbund ein absorbierendes Core - auch Sauglage genannt - ist oder allgemein in einem Core, ist das erfindungsgemäße wirkstoffdotierte wasserabsorbierende Polymerteilchen, die erfindungsgemäße Zusammensetzung oder auch der erfindungsgemäße Verbund in ein Substrat eingearbeitet. Das erfindungsgemäße wirkstoffdotierte wasserabsorbierende Polymerteilchen ist vorzugsweise zu mindestens 5 Gew.-%, bevorzugt im Bereich von 6 bis 50 Gew.-% sowie darüber hinaus bevorzugt im Bereich von 7 bis 15 Gew.-%, jeweils bezogen auf dass Core, in diesem vorhanden. In einer Gestaltung der vorliegenden Erfindung sind dem erfindungsgemäßen wirkstoffdotierten wasserabsorbierenden Polymerteilchen noch mindestens 10 Gew.-%, vorzugsweise mindestens 30 Gew.-% und besonders bevorzugt mindesten 60 Gew.-% sowie darüber hinaus bevorzugt mindestens 80 Gew.-% nicht mit Wirkstoff dotiertes wasserabsorbierende Polymerteilchen, jeweils bezogen auf die Gesamtmenge des wasserabsorbierenden Polymers im Core, beigemischt. Zudem ist es bevorzugt, dass das Core im Bereich von 40 bis 95 Gew.-%, vorzugsweise im Bereich von 50 bis 90 Gew.-% und besonders bevorzugt im Bereich von 75 bis 85 Gew.-%, jeweils bezogen auf das Core, wasserabsorbierendes Polymer, vorzugsweise als Teilchen, aufweist.

Bei diesem Substrat handelt es sich vorzugsweise um Fasermaterialien. Fasernmaterialien, die in der vorliegenden Erfindung verwendet werden können, umfassen natürlich vorkommende Fasern (modifiziert oder nicht modifiziert) als auch synthetische Fasern. Beispiele geeigneter nicht modifizierter und modifizierter natürlich vorkommender Fasern umfassen Baumwolle, Espartogras, Zuckerrohr, Grannenhaar, Flachs, Seide, Wolle, Zellstoff, chemisch modifizierter Zellstoff, Jute, Reyon, Ethylzellulose und Zelluloseacetat. Geeignete synthetische Fasern können aus Polyvinylchlorid, Polyvinylfluorid, Polytetrafluorethylen, Polyvinylidenchlorid, Polyacrylat wie Orion^{®}, Polyvinylacetat, Polyethylvinylacetat, nicht löslichem oder löslichem Polyvinylalkohol, Polyolefine, wie Polyethylen (beispielsweise PULPEX^{®}) und Polypropylenen, Polyamiden, wie Nylon, Polyestern wie DACRON^{®} oder Kodel^{®}, Polyurethanen, Polystyrolen und dergleichen hergestellt werden. Die verwendeten Fasern können nur natürlich vorkommende Fasern, nur synthetische Fasern oder irgend eine kompatible Kombination aus natürlich vorkommenden und synthetischen Fasern umfassen.

Neben den vorstehend genannten Fasermaterialien kann das Core auch thermoplastische Materialien erhalten. Beim Schmelzen wandert zumindest ein Teil dieses thermoplastischen Materials, typischerweise verursacht durch die Kapillargradienten, zwischen den Fasern hindurch zu den Kreuzungen der Fasern. Diese Kreuzungen werden zu Verbindungsstellen für das thermoplastische Material. Wenn das Element abgekühlt wird, so verfestigt sich das thermoplastische Material an diesen Kreuzungen, um Verbindungsstellen zu bilden, die die Matrix oder das Gewebe der Fasern in jeder der jeweiligen Schichten zusammen halten. Die thermoplastischen Materialien können in verschiedenen Formen, wie etwa Teilchen, Fasern oder Kombinationen aus Teilchen und Fasern, vorliegen. Diese Materialien können aus einer Vielzahl thermoplastischer Polymere ausgewählt aus Polyolefinen, wie Polyethylen (beispielsweise PULPEX^{®}) und Polypropylen, Polyestern, Copolyestern, Polyvinylacetaten, Polyethylvinylacetaten, Polyvinylchloriden, Polyvinylidenchloriden, Polyacrylaten, Polyamiden, Copolyamiden, Polystyrolen, Polyurethanen und Copolymeren der vorangehenden Stoffe, wie Vinylchlorid/Vinylacetat und dergleichen. Für Cores kommen als Substrat überwiegend aus Cellulose bestehende, vorzugsweise faserförmige Materialien in Betracht.

Das Core kann zum einen durch ein sogenanntes Airlaid-Verfahren oder durch ein sogenanntes Wetlaid-Verfahren hergestellt werden, wobei ein gemäss dem Airlaid-Verfahren hergestelltes Core bevorzugt ist. In dem Wetlaid-Verfahren werden die Fasern oder Teilchen aus absorbierendem Polymergebilde zusammen mit weiteren Substratfasem und einer Flüssigkeit zu einem Vlies verarbeitet. In dem Airlaid-Verfahren werden die Fasern oder Teilchen aus absorbierendem Polymergebilde und die Substratfasern im trockenen Zustand zu einem Vlies verarbeitet. Weitere Einzelheiten zu Airlaid-Verfahren sind in US 5,916,670 sowie US 5,866,242 und zu Wetlaid-Verfahren in US 5,300,192 beschrieben.

Ein weiterer Gegenstand der vorliegenden Erfindung ist ein Hygieneartikel, vorzugsweise Babywindeln, Damenbinden, Tampons oder Erwachsenenwindeln, besonders bevorzugt Babywindeln, beinhaltend ein erfindungsgemäßes wirkstoffdotiertes wasserabsorbierendes Polymerteilchen oder eine erfindungsgemäße Zusammensetzung oder einen erfindungsgemäßen Verbund oder mindestens zwei davon. Im allgemeinen umfasst ein als Windel eingesetzter Hygieneartikel eine wasserundurchlässige Unterschicht, eine wasserdurchlässige, vorzugsweise hydrophobe, Oberschicht und eine mindestens ein erfindungsgemäßes wirkstoffdotiertes wasserabsorbierendes Polymerteilchen oder eine erfindungsgemäße Zusammensetzung oder einen erfindungsgemäßen Verbund oder mindestens zwei davon beinhaltende Schicht, die zwischen der Unterschicht und der Oberschicht angeordnet ist. Diese Schicht ist vorzugsweise ein zuvor beschriebenes Core. Die Unterschicht kann alle dem Fachmann bekannten Materialien aufweisen, wobei Polyethylen oder Polypropylen bevorzugt sind. Die Oberschicht kann gleichfalls alle dem Fachmann bekannten und geeigneten Materialien enthalten, wobei Polyester, Polyolefine, Viskose und dergleichen bevorzugt sind, die eine so poröse Schicht ergeben, die einen ausreichenden Flüssigkeitsdurchlass der Oberschicht sicherstellen. In diesem Zusammenhang wird auf die Offenbarung in US 5,061,295, US Re. 26,151, US 3,592,194, US 3,489,148 sowie US 3,860,003 verwiesen.

Zudem ermöglicht die Erfindung die Verwendung einer erfindungsgemäßen wasserabsorbierenden Zusammensetzung zur Freisetzung eines Wirkstoffes, vorzugsweise einer Wundheilsubstanz oder einer Pflegesubstanz. Die Erfindung ermöglicht auch die Verwendung eines erfindungsgemäßen wirkstoffdotierten Polymerteilchen zur Freisetzung einer Pflegesubstanz, vorzugsweise einer Hautpflegesubstanz. Darüber hinaus betrifft die Erfindung die Verwendung eines vorstehend beschriebenen erfindungsgemäßen wasserabsorbierenden Polymers zur Freisetzung eines Wirkstoffes, vorzugsweise einer Wundheilsubstanz oder einer Pflegesubstanz aus einer Polykondensatmatrix. In diesem Zusammenhang sind insbesondere Wundverbände und Pflaster zu nennen.

Außerdem betrifft die Erfindung die Verwendung einer erfindungsgemäßen wasserabsorbierenden Zusammensetzung zur Herstellung eines Mittels zur Behandlung von einer Wunde eines höheren Wirbelorganismus oder zur Vorbeugung der Bildung einer Wunde an oder in einem höheren Wirbelorganismus. Vorzugsweise wird unter höheren Wirbelorganismus erfindungsgemäß ein Fisch, Vogel, Säugetier oder Mensch, besonders bevorzugt ein Säugetier oder Mensch und darüber hinaus bevorzugt ein Mensch verstanden. Bei den Wunden handelt es sich vorzugsweise um Schnitt-, Schürf-, Operations- oder Brandwunden sowie im Fall von Hygieneartikeln, insbesondere von Windeln, um durch langes Liegen entstehende Wunden und Hautreizungen.

Ferner ermöglicht die Erfindung ein Verfahren zur Behandlung einer Wunde eines höheren Wirbelorganismus, zur Vorbeugung der Bildung einer Wunde an oder in einem höheren Wirbelorganismus, oder zur Pflege der Haut eines höheren Wirbelorganismus, wobei die Wunde oder die Haut mindestens teilweise mit einer erfindungsgemäßen wasserabsorbierenden Zusammensetzung, einem erfindungsgemäßen wirkstoffdotierten Polymerteilchen oder einem erfindungsgemäßen Verbund abgedeckt oder in Kontakt gebracht wird.

Schließlich betrifft die Erfindung die Verwendung einer erfindungsgemäßen wasserabsorbierenden Zusammensetzung, eines erfindungsgemäßen wirkstoffdotierten Polymerteilchens oder eines erfindungsgemäßen Verbunds in einem Hygieneartikel oder einem Wundbehandlungsmittel.

Die Erfindung wird nachfolgend durch ihren Schutzumfang nichtlimitierende Beispiele näher erläutert.

### TESTVERFAHREN

### 1. ERT

Sofern nicht nachfolgend anderslautend dargestellt, werden ERT-Methoden zur Bestimmung der verschieden das wasserabsorbierende Polymer betreffenden Eigenschaften eingesetzt. "ERT" steht für EDANA Recommended Test, wobei "EDANA" für European Nonwoven And Diaper Association steht.

### 2. Extraktionstest

0,5g einer wirkstoffdotierten Probe, beispielsweise eine teilneutralisierte, schwach vernetzte Polyacrylsäure mit Dexpanthenol als Wirkstoff oder eine diese beinhaltende Polyurethanmatrix, wurden in eine 125ml Weithalsflasche auf einer Analysenwaage eingewogen. Nach Zugabe von 100ml 0,9%iger Kochsalzlösung (basierend auf destilliertem Wasser) und einem Tropfen konzentrierter Phosphorsäure wurde über eine Stunde auf dem Magnetrührer bei 350 Umdrehungen pro Minuten gerührt. Anschließend wurden 2ml der Lösung entnommen und durch einen 0,45µm Cellulosemischester-Membranfilter in ein Probenvial filtriert. Das Filtrat wurde dann einer HPLC-Analyse zugeführt, wobei die in der HPLC-Analyse verwendete Probe einen sauren pH-Wert im Bereich von 2,5 bis 3,0 aufwies.

Der Gehalt an Wirkstoff wurde an Hand der HPLC-Analyseergebnisse durch externe Kalibrierung bestimmt. Hierzu wurde der zu bestimmende Wirkstoff mittels Analysenwaage in einem 100ml fassenden Messkolben mit mindestens 10mg auf 0,1mg genau eingewogen. Anschließend wurde der Messkolben mit ultrareinem Wasser bis zur Marke aufgefüllt. Entsprechend der Konzentration der entstandenen Stammlösung wird nun auf der Analysewaage eine Verdünnungsreihe hergestellt. Mittels dieser Verdünnungsreihe wird eine Kalibrierkurve durch HPLC-Analysen erstellt. Die Menge des über eine Stunde extrahierten Wirkstoffs wird durch den Vergleich der HPLC-Analyseergebnisse des entsprechenden Wirkstoffs mit der Kalibrierkurve ermittelt.

Die Chromatographischen Bedingungen wurden in Abhängigkeit des zu bestimmenden Wirkstoffs optimiert. Im Fall des Dexpanthenols wurde eine Säule des Typs GromSil 300 ODS-5 5µm (250 x 4mm) verwendet. Das Eluent wurde hergestellt, indem 13,61g KH₂PO₄ in ein 31 fassendes Becherglas eingewogen und nach Zugabe von 2000ml ultrareinem Wasser gelöst wurden. Anschließend wurde mit konzentrierter Phosphorsäure ein pH-Wert von 2,5 bis 3,0 eingestellt. Im Fall von Dexpanthenol wurde ein Fluss von 0,8ml/min. eingestellt. Die Injektion erfolgte über ein Loop von 20µl.

### 3. Rheologische Charakterisierung

Aus einem Pflaster wird mittig eine Probe mit einem Durchmesser von 8mm ausgestanzt und eine Stunde bei 23±2°C und 50±5%r.F. vorkonditioniert. Die Probe wird mittig auf einem 8mm Platte Drehkörper aufgeklebt und an einem schubspannungsgesteuerten Rheometermit mit einem Peltierelement zur Temperierung (z.B. RS-75 von HAAKE) vermessen. Dazu wird die Probe mit einer Normalkraft von 1,3N auf die untere Platte gedrückt. Nach einer Konditionierung von 5 Minuten bei 25±0,2°C werden bei einer Schubspannung von 700Pa die viskoelastischen Eigenschaften (Speicher- und Verlustmodul) im Frequenzbereich von ω= 0,3 bis 30 rad/s bestimmt. Der tanδ wird aus dem Quotienten aus Verlust- und Speichermodul berechnet.

### 4. Flüssigkeitsaufnahme

Aus einem Pflaster wird mittig eine Probe mit einem Durchmesser von 15 mm ausgestanzt und eine Stunde bei 23 ± 2 °C und 50 ± 5 % r.F. vorkonditioniert. Die Proben werden gewogen und für 3 Stunden vollständig in physiologische 23 ± 0,5 °C warme Natriumchloridlösung getaucht. Die Proben werden erneut gewogen und die Flüssigkeitsaufnahme aus der Wägedifferenz berechnet.

### 5. Wasserdampfdurchlässigkeit

Die Prüfung erfolgt nach ASTM E 96 (water method), mit folgenden Abweichungen: Die Öffnung des Prüfgefäßes beträgt 804 mm². Das Material wird 24 Std. vorkonditioniert bei 23 ± 2 °C und 50 ± 5 % r.F. Der Abstand zwischen Wasserspiegel im Prüfgefäß und der Probe beträgt 35 ± 5 mm Das Rückwiegen der mit Proben bestückten Prüfgefäße erfolgt nach 24 Std., in denen diese im Klimaschrank bei 37 ± 1,5 °C und 30 ± 3 % r.F. gelagert werden.

### 6. O₂-Durchlässigkeit

Prüfung wurde gemäß ASTM D3985-81 durchgeführt.

### BEISPIELE

### 1. HERSTELLUNG VON DEXPANTHENOL-DOTIERTER WASSERABSORBIERENDER POLYMER ALS WASSERABSORBIERENDE POLYMERZUSAMMENSETZUNG

### Variante A: Zusatz des Dexpanthenols in Monomerlösung

In 944,77 g einer wässrigen Lösung von Natriumacrylat mit einem Neutralisationsgrad von 70 Mol-% (Monomer-Konzentration des Ansatzes 36,4%) wurde 0,45 g Triallylamin als Vernetzer gelöst. Anschließend wurde 50 g einer 75%igen Lösung von Dexpanthenol in Wasser zugegeben. Die Monomerenlösung wurde in einem Kunststoff-Polymersisationsgefäß für 30 Minuten mit Stickstoff durchspült, um den darin gelösten Sauerstoff zu entferne. Bei einer Temperatur von 4°C wurde die Polymerisation durch die aufeinanderfolgende Zugabe von 0,25 g Natriumperoxidisulfat in 10 g dest. Wasser, 0,1 g 2,2'-Azobis-2-amidinopropandihydro-chlorid in 10 g dest. Wasser, 0,2 g tertier-Buthylhydroperoxid in 10 g dest. Wasser und 0,015 Ascorbinsäure in 2 g dest. Wasser gestartet. Nachdem die Endtemperatur (100°C) erreicht war, wurde das Gel mit einem Fleischwolf zerkleinert und in einem Umluftofen getrocknet (Trocknungstemperatur und -dauer sind in der nachfolgenden Tabelle angegeben). Das getrocknete Produkt wurde grob zer-stoßen, gemahlen und die Partikel der Größe 150 - 850 µm zur weiteren Verabreitung ausgesiebt. Zur Einarbeitung des absorbierenden Polymers in eine Polyurethan-Matrix wurde das absorbierende Polymer-Pulver auf eine Partikelgröße von < 300 µm gemahlen. Die so erhaltenen wasserabsorbierenden Polymere weisen eine Restfeuchte von 3 Gew.-%, bezogen auf das wasserabsorbierende Polymer auf.

### Variante B: Zusatz des Dexpanthenols zum Polymergel

In 993,08 g einer wässrigen Lösung von Natriumacrylat mit einem Neutralisationsgrad von 70 Mol-% (Monomer-Konzentration des Ansatzes 36,4%) wurde 0,45 g Triallylamin als Vernetzer gelöst. Die Monomerenlösung wurde in einem Kunststoff-Polymerisationsgefäß für 30 Minuten mit Stickstoff gespült, um den gelösten Sauerstoff zu entfernen. Bei einer Temperatur von 4°C wurde die Polymerisation durch die aufeinanderfolgende Zugabe von 0,25 g Natriumperoxidisulfat in 10 g dest. Wasser, 0,1 g 2,2'-Azobis-2-amidinopropandihydrochlorid in 10 g dest. Wasser, 0,2 g tertier-Buthylhydroperoxid in 10 g dest. Wasser und 0,015 Ascorbinsäure in 2 g dest. Wasser gestartet. Nachdem die Endtemperatur (ca. 100°C) erreicht war, wurde das Gel mit einem Fleischwolf zerkleinert und mit 50 g einer 75%igen Lösung von Dexpanthenol in Wasser gleichmäßig besprüht und vermischt. Anschließend wurde in einem Umluftofen getrocknet (Trocknungstemperatur und -dauer sind der Tabelle zu entnehmen) das getrocknete Produkt wurde grob zerstoßen, gemahlen und die Partikel der Größe von 150 bis 180 µm zur weiteren Verarbeitung ausgesiebt. Zur Einarbeitung des Superabsorberpulver in eine Polyurethan-Matrix wurde das Superabsorberpulver auf eine Partikelgröße von < 300 µm gemahlen. Die so erhaltenen wasserabsorbierenden Polymere weisen eine Restfeuchte von 3 Gew.-%, bezogen auf das wasserabsorbierende Polymer auf.

Die Restfeuchte der in der folgenden Tabelle 1 aufgeführten wasserabsorbierenden Polymere lag bei 3 %.

**Tabelle 1**

| | Dexpanthenol-Zusatz | Trocknung °C / min. | Lösliche Anteil 16 h [%] | CRC [g/g] | AAP [g/g] | Restmon. gehalt [ppm] | Extrahierbares Dex-panthenol [%]⁴ |
|---|---|---|---|---|---|---|---|
| Beispiel 1 | 10%¹ (Variante A) | 120 / 210 | 11,9 | 34,0 | 7 | 145 | 80 |
| | 50 g² 75%³ Lösung | | | | | | |
| Beispiel 2 | 10% (Variante B) | 120 / 210 | 12,1 | 35,2 | 7,2 | 210 | 91 |
| | 50g 75% Lösung | | | | | | |
| Beispiel 3 | 5% (Variante A) | 110 / 270 | 12,4 | 33,7 | 8 | 160 | 86 |
| | 25g 75% Lösung | | | | | | |
| Beispiel 4 | 5% (Variante B) | 120 / 210 | 12,3 | 33,7 | 7,8 | 155 | 98 |
| | 25g 75% Lösung | | | | | | |

| | | | | | | | |
|---|---|---|---|---|---|---|---|
| Prozentualer Anteil Dexpanthenol in Superabsorberpulver ² Menge der eingesetzten Dexpanthenol-Lösung ³ Konzentration der Dexpanthenol-Lösung ⁴ Gemäß des Extraktionstest nach 1 Stunde extrahierter Anteil des ursprünglich in dem Superabsorberpulver eingesetzten Dexpanthenols | | | | | | | |

### 2. HERSTELLUNG EINER WUNDAUFLAGE MIT DEXPANTHENOL-DOTIERTEM SUPERABSORBER

### 2.1 Vorbereitung der beiden Komponenten:

**Tabelle 2**

| Gew.-% | |
|---|---|
| Komponente 1 | |
| 82 | Polyether-Polyol* |
| 9 | Isocyanat-Prepolymer** |
| 9 | Absorbierendes Polymer nach Beispiel 1, dotiert mit 10% Dexpanthenol |

| Komponente 2 | |
|---|---|
| 90 | Polyether-Polyol* |
| 10 | Katalysator*** |

| | |
|---|---|
| * Pentaerythrit + Propylenoxid + Ethylenoxid - Mischpolymerisat mit Ethylenoxid-Endblock Funktionalität: 4, OH-Zahl: 35, mittleres Molgewicht: 6400 (berechnet), Viskosität (23°C): 1000 mPas, Ethylenoxid-Gehalt: 20 Gew.-% ** NCO-terminiertes Prepolymer aus Umsatz bei 80 °C von Hexamethylendiisocyanat und Polypropylenglykol (mittleres Molekulargewicht: 220) im Molverhältnis 5:1 und anschließender Vakuumdestillation bei ca. 0,5 mbar (50 Pa) bis auf einen HDI-Monomer-Restgehalt < 0,5 Gew.-% NCO-Gehalt: 12,6 Gew.-%, Viskosität (23 °C): 5000 mPas *** Lösung von 1 mol des Bi(III)Salzes mit 2,2-Dimethyloctansäure in 3 mol 2,2-Dimethyloctansäure (Wismutgehalt ca. 17 Gew.-%) | |

Die in Tabelle 2 angegebenen Mengen wurden eingewogen und 24 Stunden auf einem Rollboclc gemischt.

### 2.2 Herstellung von Ausstrichen

98 Gew.-% der Komponente 1 und 2 Gew.-% der Komponente 2 wurden über etwa 40 Sekunden von Hand gleichmäßig vermischt, auf ein Trennpapier gegeben und mittels eines Streichbalkens mit einer Spalteinstellung von 1 mm zwischen dem Trennpapier und einer Kunststofffolie mit einer Wasserdampfdurchlässigkeit von 750 g/(m²×24h) ausgestrichen, um anschließend über 5 Minuten in einem Trockenschrank bei 60 °C abzureagieren. Der so erhaltene Ausstrich besitzt ein Flächengewicht von 850 g/m² und kann in für Wundauflagen oder Eigenschafts-messungen geeignete Formen zu geschnitten oder ausgestanzt werden. Die Eigenschaften des Ausstrichs sind in Tabelle 3 angegeben.

**Tabelle 3**

| Eigenschaft | Werte^{#} |
|---|---|
| Viskoselastizität | [tanδ (ω=0,3 rad/s)] |
| | 0,225 |
| Flüssigkeitsaufnahme | [g/100cm²] |
| | 12,5 |
| Wasserdampfdurchlässigkeit | [g/(m²×24h)] |
| | 350 |
| O₂-Durchlässigkeit | [cm³/(m²×24h)] |
| | 2050 |

| | |
|---|---|
| ^{#} Mittelwerte von Mehrfachbestimmungen | |

## Patentansprüche

1. Wirkstoffdotierte wasserabsorbierende Polymerteilchen, beinhaltend
Φ 1. als Wirkstoff eine Pflegesubstanz oder eine Wundheilsubstanz in einer Menge im Bereich von 0,001 bis 30 Gew.-%, bezogen auf die wirkstoffdotierten wasserabsorbierende Polymerteilchen,
Φ2. eine Absorbermatrix in einer Menge im Bereich von 70 bis 99,999 Gew.-%, bezogen auf die wirkstoffdotierten wasserabsorbierende Polymerteilchen,
wobei die Absorbermatrix zu mindestens 90 Gew.-%, bezogen auf die Absorbermatrix, aus einer vernetzten Polyacrylsäure besteht,
wobei die vernetzte Polyacrylsäure zu mindestens 90 Gew.-%, bezogen auf die vernetzte Polyacrylsäure, aus einer zu mindestens 30 Mol-% teilneutralisierten Acrylsäure besteht.

2. Wirkstoffdotiertes wasserabsorbierendes Polymerteilchen nach Anspruch 1, wobei der Wirkstoff über die gesamte Absorbermatrix verteilt ist.

3. Wirkstoffdotiertes wasserabsorbierendes Polymerteilchen nach einem der vorhergehenden Ansprüche, wobei diese einen Gehalt an restlichem Monomeren, auf dem das wasserabsorbierende Polymerteilchen basiert, unter 500 ppm zeigt.

4. Wirkstoffdotiertes wasserabsorbierendes Polymerteilchen nach einem der vorhergehenden Ansprüche, wobei dieses eine Wirkstoffverfügbarkeit von mindestens 40 Gew.-% nach dem hierin angegebenen Extraktions-Test zeigt.

5. Wasserabsorbierende Zusammensetzung, beinhaltend
Γ1. eine Polykondensatmatrix, basierend auf mindestens einem Polykondensatmonomer mit mindestens einer Polykondensatgruppe, und
Γ2. ein in einem der Ansprüche 1 bis 4 definiertes wirkstoffdotiertes wasserabsorbierendes Polymerteilchen,
wobei das teilchenförmige wasserabsorbierende Polymer mindestens teilweise von der Polykondensatmatrix umgeben ist,
wobei mindestens das teilchenförmige wasserabsorbierende Polymer den Wirkstoff aufweist, und wobei die wasserabsorbierende Zusammensetzung eine Wirkstoffverfügbarkeit von mindestens 10 Gew.-% nach dem hierin angegebenen Extraktions- Test zeigt,
und wobei die wasseraborbierende Zusammensetzung erhältlich ist durch ein Verfahren, bei dem das den Wirkstoff beinhaltende teilchenförmige wasserabsorbierende Polymer vor Abschluss der Polykondensatmatrixbildung mit dem Polykondensatmonomer in Kontakt gebracht wird.

6. Verfahren zu Herstellung einer wasserabsorbierenden Zusammensetzung, wobei ein in einem der Ansprüche 1 bis 4 definiertes wirkstoffdotiertes wasserabsorbierendes Polymerteilchen mindestens teilweise in eine auf mindesten einem Polykondensatmonomer basierenden Kondensatmatrix eingearbeitet wird, wobei das in einem der Ansprüche 1 bis 4 definierte wirkstoffdotierte wasserabsorbierende Polymerteilchen mit dem Polykondensatmonomer vor Abschluss der Polykondensatmatrixbildung in Kontakt gebracht wird.

7. Wasserabsorbierende Zusammensetzung, erhältlich nach einem Verfahren nach Anspruch 6.

8. Zusammensetzung nach Anspruch 5 oder 7, wobei das wasserabsorbierende Polymer mindestens eine der nachfolgenden Eigenschaften aufweist:
A1) eine Partikelgrößenverteilung aufweisen, wobei mindestens 80 Gew.- % der Partikel eine Partikelgröße in einem Bereich von 20 µm bis 900 µm nach ERT 420.1 -99 besitzen;
A2) eine Centrifuge Retention Capacity (CRC) von mindestens 10 g/g, vorzugsweise mindestens 20 g/g nach ERT 441.1 -99;
A3) eine Absorption Against Pressure (AAP) bei 49 g/cm2 von mindestens 4 g/g nach ERT 442-1.99;
A4) einen Gehalt an wasserlöslichem Polymer nach 16 Stunden Extraktion von weniger als 25 Gew.-%, jeweils bezogen auf das Gesamtgewicht des wasserabsorbierenden Polymers, nach ERT 470.1 - 99,
A5) eine Restfeuchtigkeit von höchstens 15 Gew.-%, jeweils bezogen auf das Gesamtgewicht des wasserabsorbierenden Polymers, nach ERT 430.1-99.

9. Zusammensetzung nach einem der Ansprüche 5, 7 oder 8, wobei die Polykondensatmatrix mindestens 10 Gew.-%, bezogen auf die Polykondensatmatrix, ein Polyurethan aufweist.

10. Zusammensetzung nach einem der Ansprüche 5, 7 bis 9, wobei die Polykondensatmatrix als Schaum vorliegt.

11. Verbund, beinhaltend eine Zusammensetzung nach einem der Ansprüche 5, 7 bis 10.

12. Verbund nach Anspruch 11, mit mindestens einer der folgenden Eigenschaften:
V1) einer Viskoselastizität [tanδ (w = 0,3 rad/s)] im Bereich von 0,1 bis 10;
V2) einer Flüssigkeitsaufhahme [g/ 100cm2] im von mindestens 5;
V3) einer Wasserdampfdurchlässigkeit [g/(m2x24h)] von mindestens 100; oder
V4) einer 02-Durchlässigkeit [cm3/(m2x24h)] von mindestens 100.

13. Verbund nach Anspruch 11 oder 12, zusätzlich neben der Zusammensetzung nach einem der Ansprüche 5, 7 bis 10 beinhaltend einen Film.

14. Verbund nach Anspruch 13, wobei der Film eine Wasserdampfdurchlässigkeit [g/(m *24h)] im Bereich von 100 bis 2000 besitzt.

15. Verbund nach Anspruch 13 oder 14, wobei die Zusammensetzung unmittelbar an die Folie angrenzt.

16. Hygieneartikel, beinhaltend ein wirkstoffdotiertes wasserabsorbierendes Polymerteilchen nach einem der Ansprüche 1 bis 4 oder eine Zusammensetzung nach einem der Ansprüche 5, 7 bis 10 oder einen Verbund nach einem Ansprüche 11 bis 15 oder mindestens zwei davon.

17. Verwendung einer Zusammensetzung nach einem der Ansprüche 5, 7 bis 10 zur Herstellung eines Mittels zur Freisetzung einer Wundheilsubstanz.

18. Verwendung eines in einem der Ansprüche 1 bis 4 definierten wirkstoffdotierten wasserabsorbierenden Polymerteilchens zur Herstellung eines Mittels zur Freisetzung einer Wundheilsubstanz aus einer Polykondensatmatrix, wobei das die Wundheilsubstanz beinhaltende teilchenförmige wasserabsorbierende Polymer vor Abschluss der Polykondensatmatrixbildung mit dem Polykondensatmonomer in Kontakt gebracht wird.

19. Verwendung eines wirkstoffdotierten wasserabsorbierenden Polymerteilchens nach einem der Ansprüche 1 bis 4 oder einer Zusammensetzung nach einem der Ansprüche 5, 7 bis 10 oder eines Verbunds nach einem Ansprüche 11 bis 15 oder mindestens zwei davon zur Herstellung eines Mittels zur Behandlung von einer Wunde eines höheren Wirbelorganismus oder zur Vorbeugung der Bildung einer Wunde an oder in einem höheren Wirbelorganismus.

20. Verwendung eines wirkstoffdotierten wasserabsorbierenden Polymerteilchens nach einem der Ansprüche 1 bis 4 einer Zusammensetzung nach einem der Ansprüche 5, 7 bis 10 oder einen Verbund nach einem der Ansprüche 11 bis 15 oder mindestens zwei davon in einem Hygieneartikel oder zur Herstellung eines Wundbehandlungsmittels.

## Claims

1. Water-absorbent polymer particles doped with active ingredient, comprising
Φ1. as active ingredient, a care substance or a wound healing substance in an amount in the range from 0.001 to 30% by weight, based on the water-absorbent polymer particles doped with active ingredient,
Φ2. an absorber matrix in an amount in the range from 70 to 99.999% by weight, based on the water-absorbent polymer particles doped with active ingredient,
where the absorber matrix consists to at least 90% by weight, based on the absorber matrix, of a crosslinked polyacrylic acid,
where the crosslinked polyacrylic acid consists to at least 90% by weight, based on the crosslinked polyacrylic acid, of an acrylic acid partially neutralized to at least 30 mol%.

2. Water-absorbent polymer particle doped with active ingredient according to Claim 1, where the active ingredient is distributed over the entire absorber matrix.

3. Water-absorbent polymer particle doped with active ingredient according to one of the preceding claims, where this exhibits a content of residual monomer on which the water-absorbent polymer particle is based of less than 500 ppm.

4. Water-absorbent polymer particle doped with active ingredient according to one of the preceding claims, where this exhibits an active ingredient availability of at least 40% by weight according to the extraction test stated herein.

5. Water-absorbent composition comprising
Γ1. a polycondensate matrix based on at least one polycondensate monomer with at least one polycondensate group, and
Γ2. a water-absorbent polymer particle doped with active ingredient as defined in one of Claims 1 to 4,
where the particulate water-absorbent polymer is surrounded at least partially by the polycondensate matrix,
where at least the particulate water-absorbent polymer has the active ingredient, and
where the water-absorbent composition exhibits an active ingredient availability of at least 10% by weight according to the extraction test stated herein,
and where the water-absorbent composition is obtainable by a process in which the particulate water-absorbent polymer comprising the active ingredient is brought into contact with the polycondensate monomer before the polycondensate matrix formation has concluded.

6. Process for producing a water-absorbent composition, where a water-absorbent polymer particle doped with active ingredient as defined in one of Claims 1 to 4 is incorporated at least partially into a condensate matrix based on at least one polycondensate monomer, where the water-absorbent polymer particle doped with active ingredient as defined in one of Claims 1 to 4 is brought into contact with the polycondensate monomer before the polycondensate matrix formation has concluded.

7. Water-absorbent composition obtainable by a process according to Claim 6.

8. Composition according to Claim 5 or 7, where the water-absorbent polymer has at least one of the following properties:
A1) a particle size distribution where at least 80% by weight of the particles have a particle size in a range form 20 µm to 900 µm according to ERT 420.1-99;
A2) a centrifuge retention capacity (CRC) of at least 10 g/g, preferably at least 20 g/g according to ERT 441.1-99;
A3) an absorption against pressure (AAP) at 49 g/cm² of at least 4 g/g according to ERT 442.1-99;
A4) a content of water-soluble polymer after extraction for 16 hours of less than 25% by weight, in each case based on the total weight of the water-absorbent polymer, according to ERT 470.1-99,
A5) a residual moisture of at most 15% by weight, in each case based on the total weight of the water-absorbent polymer, according to ERT 430.1-99.

9. Composition according to one of Claims 5, 7 or 8, where the polycondensate matrix has at least 10% by weight, based on the polycondensate matrix, of a polyurethane.

10. Composition according to one of Claims 5, 7 to 9, where the polycondensate matrix is in the form of a foam.

11. Composite comprising a composition according to one of Claims 5, 7 to 10.

12. Composite according to Claim 11, having at least one of the following properties:
V1) a viscoelasticity [tanδ (ω = 0.3 rad/s)] in the range from 0.1 to 10;
V2) a liquid absorption [g/100 cm²] of at least 5;
V3) a water vapour permeability [g/(m²×24h)] of at least 100; or
V4) an O₂ permeability [cm³/(m²×24h)] of at least 100.

13. Composite according to Claim 11 or 12, additionally comprising a film in addition to the composition according to one of Claims 5, 7 to 10.

14. Composite according to Claim 13, where the film has a water vapour permeability [g/(m*24h)] in the range from 100 to 2000.

15. Composite according to Claim 13 or 14, where the composition directly adjoins the foil.

16. Hygiene article comprising a water-absorbent polymer particle doped with active ingredient according to one of Claims 1 to 4 or a composition according to one of Claims 5, 7 to 10 or a composite according to one of Claims 11 to 15 or at least two thereof.

17. Use of a composition according to one of Claims 5, 7 to 10 for producing a means for releasing a wound healing substance.

18. Use of a water-absorbent polymer particle doped with active ingredient as defined in one of Claims 1 to 4 for producing a means for releasing a wound healing substance from a polycondensate matrix, where the particulate water-absorbent polymer comprising the wound healing substance is brought into contact with the polycondensate monomer before the polycondensate matrix formation has concluded.

19. Use of a water-absorbent polymer particle doped with active ingredient according to one of Claims 1 to 4 or of a composition according to one of Claims 5, 7 to 10 or of a composite according to one of Claims 11 to 15 or at least two thereof for producing a means for treating a wound of a higher vertebrate or for preventing the formation of a wound on or in a higher vertebrate.

20. Use of a water-absorbent polymer particle doped with active ingredient according to one of Claims 1 to 4 or of a composition according to one of Claims 5, 7 to 10 or of a composite according to one of Claims 11 to 15 or at least two thereof in a hygiene article or for producing a wound treatment means.

## Revendications

1. Particules polymères hydroabsorbantes dopées en principe actif, contenant
Φ1. en tant que principe actif, une substance de soin ou une substance cicatrisante en une quantité comprise dans la plage de 0,001 à 30 % en poids par rapport aux particules polymères hydroabsorbantes dopées en principe actif,
Φ2. une matrice absorbante en une quantité comprise dans la plage de 70 à 99,999 % en poids par rapport aux particules polymères hydroabsorbantes dopées en principe actif,
dans lesquelles la matrice absorbante est, pour au moins 90 % en poids par rapport à la matrice absorbante, constituée d'un poly(acide acrylique) réticulé,
le poly(acide acrylique) réticulé étant, pour au moins 90 % en poids par rapport au poly(acide acrylique) réticulé, constitué d'un acide acrylique partiellement neutralisé à au moins 30 % en moles.

2. Particule polymère hydroabsorbante dopée en principe actif selon la revendication 1, dans laquelle le principe actif est réparti sur la totalité de la matrice absorbante.

3. Particule polymère hydroabsorbante dopée en principe actif selon l'une des revendications précédentes, qui présente une teneur inférieure à 500 ppm en un monomère résiduel sur lequel se base la particule polymère hydroabsorbante.

4. Particule polymère hydroabsorbante dopée en principe actif selon l'une des revendications précédentes, qui présente une disponibilité du principe actif d'au moins 40 % en poids d'après le test d'extraction présenté dans la présente invention.

5. Composition hydroabsorbante, contenant
Γ1. une matrice de polycondensat, se basant sur au moins un monomère de polycondensation ayant au moins un groupe polycondensat, et
Γ2. une particule polymère hydroabsorbante dopée en principe actif, définie dans l'une des revendications 1 à 4,
dans laquelle le polymère hydroabsorbant particulaire est au moins partiellement entouré par la matrice de polycondensat,
au moins le polymère hydroabsorbant particulaire comportant le principe actif, et
la composition hydroabsorbante présentant une disponibilité du principe actif d'au moins 10 % en poids, d'après le test d'extraction présenté dans la présente invention,
et la composition hydroabsorbante pouvant être obtenue par un procédé dans lequel le polymère hydroabsorbant particulaire contenant le principe actif est, avant la fin de la formation de la matrice de polycondensat, mis en contact avec le monomère de polycondensation.

6. Procédé de fabrication d'une composition hydroabsorbante, dans lequel une particule polymère hydroabsorbante dopée en principe actif, définie dans l'une des revendications 1 à 4, est au moins partiellement incorporée dans une matrice de condensat se basant sur au moins un monomère de polycondensation, la particule polymère hydroabsorbante dopée en principe actif, définie dans l'une des revendications 1 à 4, étant mise en contact avec le monomère de polycondensation avant la fin de la formation de la matrice de polycondensat.

7. Composition hydroabsorbante pouvant être obtenue par un procédé selon la revendication 6.

8. Composition selon la revendication 5 ou 7, dans laquelle le polymère hydroabsorbant présente au moins l'une des propriétés suivantes :
A1) une distribution granulométrique pour laquelle au moins 80 % en poids des particules ont une granulométrie comprise dans la plage de 20 µm à 900 µm selon ERT 420.1-99 ;
A2) une capacité de rétention à la centrifugeuse (CRC) d'au moins 10 g/g, de préférence d'au moins 20 g/g selon ERT 441.1-99 ;
A3) une absorption sous pression (AAP), pour 49 g/cm², d'au moins 4 g/g selon ERT 442.1-99 ;
A4) une teneur en polymère hydrosoluble, après 16 heures d'extraction, d'au moins 25 % en poids, dans chaque cas par rapport au poids total du polymère hydroabsorbant, selon ERT 470.1-99 ;
A5) une humidité résiduelle d'au plus 15 % en poids, dans chaque cas par rapport au poids total du polymère hydroabsorbant, selon ERT 430.1-99.

9. Composition selon l'une des revendications 5, 7 ou 8, dans laquelle la matrice de polycondensat comprend au moins 10 % en poids, par rapport à la matrice de polycondensat, d'un polyuréthanne.

10. Composition selon l'une des revendications 5, 7 à 9, dans laquelle la matrice de polycondensat se présente sous forme d'une mousse.

11. Pansement contenant une composition selon l'une des revendications 5, 7 à 10.

12. Pansement selon la revendication 11, présentant au moins l'une des propriétés suivantes :
V1) une élasticité retardée [tanδ(ω=0,3 rad/s)] comprise dans la plage de 0,1 à 10 ;
V2) une capacité d'absorption des liquides [g/100 cm²] d'au moins 5 ;
V3) une perméabilité à la vapeur d'eau [g/(m²x24h)] d'au moins 100 ; ou
V4) une perméabilité à O₂ [cm³/(m²x24h)] d'au moins 100.

13. Pansement selon la revendication 11 ou 12, contenant un film en plus de la composition selon l'une des revendications 5, 7 à 10.

14. Pansement selon la revendication 13, dans lequel le film présente une perméabilité à la vapeur d'eau [g/(m*24h)] comprise dans la plage de 100 à 2000.

15. Pansement selon la revendication 13 ou 14, dans lequel la composition est contiguë à la feuille.

16. Produit hygiénique, contenant une particule polymère hydroabsorbante dopée en principe actif selon l'une des revendications 1 à 4 ou une composition selon l'une des revendications 5, 7 à 10 ou un pansement selon l'une des revendications 11 à 15 ou au moins deux d'entre eux.

17. Utilisation d'une composition selon l'une des revendications 5, 7 à 10 pour la fabrication d'un produit destiné à la libération d'une substance cicatrisante.

18. Utilisation d'une particule polymère hydroabsorbante dopée en principe actif définie dans l'une des revendications 1 à 4, pour la fabrication d'un produit destiné à la libération d'une substance cicatrisante à partir d'une matrice de polycondensat, le polymère hydroabsorbant particulaire contenant la substance cicatrisante étant, avant la fin de la formation de la matrice de polycondensat, mis en contact avec le monomère de polycondensation.

19. Utilisation d'une particule polymère hydroabsorbante dopée en principe actif selon l'une des revendications 1 à 4 ou d'une composition selon l'une des revendications 5, 7 à 10 ou d'un pansement selon l'une des revendications 11 à 15 ou d'au moins deux d'entre eux pour la fabrication d'un produit destiné au traitement d'une plaie d'un organisme vertébré supérieur ou à la prévention de la formation d'une plaie sur ou dans un organisme vertébré supérieur.

20. Utilisation d'une particule polymère hydroabsorbante dopée en principe actif selon l'une des revendications 1 à 4 ou d'une composition selon l'une des revendications 5, 7 à 10 ou d'un pansement selon l'une des revendications 11 à 15 ou d'au moins deux d'entre eux dans un article hygiénique, ou pour la fabrication d'un produit pour le traitement des plaies.
